(19) Europäisches Patentamt
European Patent Office
Office européen des brevets

(11) EP 4 289 811 A1

# (12) EUROPEAN PATENT APPLICATION

(43) Date of publication:
**13.12.2023 Bulletin 2023/50**

(21) Application number: **22178410.1**

(22) Date of filing: **10.06.2022**

(51) International Patent Classification (IPC):
***C07C 47/575*** *(2006.01)* ***C07C 69/92*** *(2006.01)*

(52) Cooperative Patent Classification (CPC):
(C-Sets available)
**C07C 69/92; C07C 47/575; C07C 201/12** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **École Polytechnique Fédérale de Lausanne (EPFL)**
**1015 Lausanne (CH)**

(72) Inventors:
- **LIU, Mingyang**
  **1020 Renens (CH)**
- **DYSON, Paul**
  **1024 Ecublens (CH)**

(74) Representative: **Cohausz & Florack Patent- & Rechtsanwälte Partnerschaftsgesellschaft mbB**
**Bleichstraße 14**
**40211 Düsseldorf (DE)**

## (54) DIARYL ETHER COMPOUND AND MANUFACTURING METHOD FROM LIGNIN

(57) Herein described are diaryl ether compounds having the structure (I)

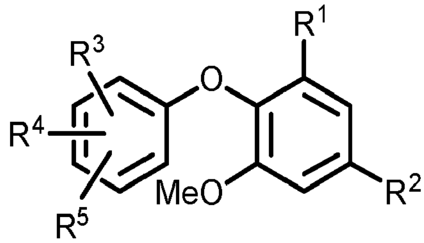

(I)

as well as a method for their manufacture from lignin.

EP 4 289 811 A1

(52) Cooperative Patent Classification (CPC): (Cont.)

C-Sets
**C07C 201/12, C07C 205/38**

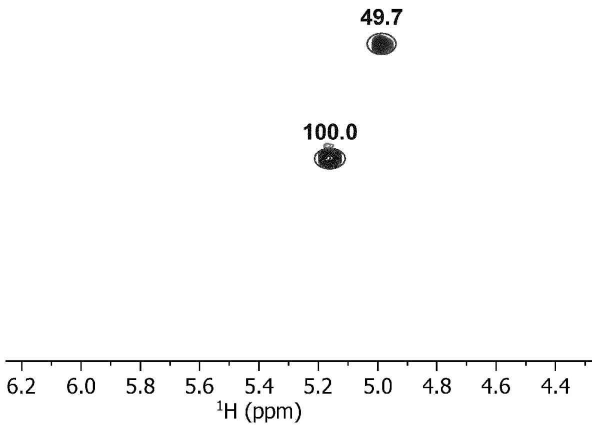


Fig.1a

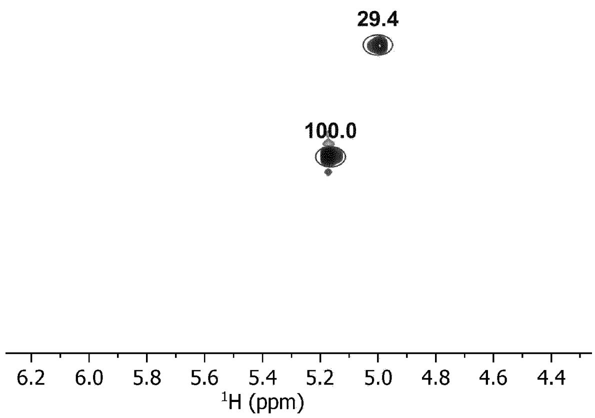


Fig.1b

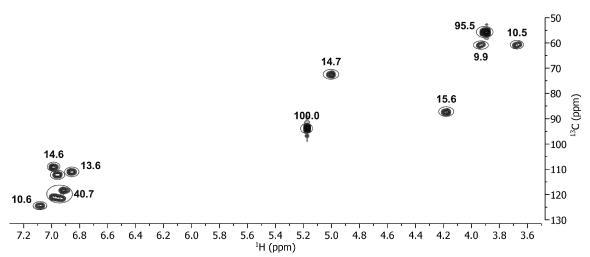


Fig.1c

## Description

The invention relates to a diaryl ether compound and a method for the manufacture of the diaryl ether compound from lignin.

BACKGROUND

Lignin is an amorphous polymer that comprises aromatic repeat units. Lignin contains the following repeat units and may contain other repeat units

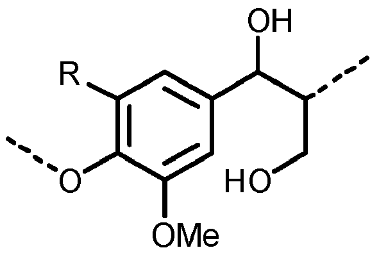

R = H or OMe

.

Lignin is a sustainable source of aromatic compounds and is available in abundant quantities as a waste product from the pulp and paper and bioethanol industries (see e.g. Ragauskas A. J., Beckham G. T., Biddy M. J., Chandra R., Chen F., Davis M. F., Davison B. H., Dixon R. A., Gilna P., Keller M., Langan P., Naskar A. K., Saddler J. N., Tschaplinski T., J., Tuskan G., A., Wyman C., E., Science 2014, 344, 1246843; Rinaldi R., Jastrzebski R., Clough M. T., Ralph J., Kennema M.; Bruijnincx P. C. A., Weckhuysen B. M., Angew. Chem. Int. Ed. 2016, 55, 8164-8215; Sun Z., Fridrich B., de Santi A., Elangovan S., Barta K., Chem. Rev. 2018, 118, 614-678; Schutyser W., Renders T., Van den Bosch S., Koelewijn S. F., Beckham G. T., Sels B. F., Chem. Soc. Rev. 2018, 47, 852-908; Questell-Santiago Y. M., Galkin M. V., Barta K., Luterbacher J. S., Nat. Rev. Chem. 2020, 4, 311-330; Abu-Omar M. M., Barta K., Beckham G. T., Luterbacher J. S., Ralph J., Rinaldi R., Roman-Leshkov Y., Samec J. S. M., Sels B. F., Wang F., Energy Environ. Sci. 2021, 14, 262-292).

Nonetheless, lignin is under-exploited as a renewable chemical feedstock due to the limited number of efficient and selective downstream processing strategies available.

Various methods have been extensively studied for the conversion of lignin into aromatic products that can broadly be classified as catalytic oxidative degradation, catalytic reductive degradation and acid/base-catalyzed degradation. Catalytic oxidative degradation has a number of advantages compared to other catalytic fractionation methods including hydrogenolysis (a reductive process), and acid/base-catalyzed degradation (see e.g. Questell-Santiago Y. M., Galkin M. V., Barta K., Luterbacher J. S., Nat. Rev. Chem. 2020,4, 311-330; Behling R., Valange S., Chatel G., Green Chem. 2016, 18, 1839-1854; Yang C., Maldonado S., Stephenson C. R. J., ACS Catal. 2021, 11, 10104-10114). Catalytic oxidative degradation advantageously takes place under mild and environmentally benign conditions, which contrasts with hydrogenolysis that uses noble metal catalysts, high reaction temperatures and pressures, or the use of corrosive acid/base-catalyzed degradation reagents. In addition, catalytic oxidative degradation has the potential to retain key functionality in the products that could be relevant in subsequent synthetic steps (see e.g. Cai Z., Long J., Li Y., Ye L., Yin B., France L. J., Dong J., Zheng L., He H., Liu S., Tsang S. C. E., Li X., Chem 2019, 5, 2365-2377; Luo H., Weeda E. P., Alherech M., Anson C. W., Karlen S. D., Cui Y., Foster C. E., Stahl S. S., J. Am. Chem. Soc. 2021, 143, 15462-15470; Subbotina E., Rukkijakan T., Marquez-Medina M. D., Yu X., Johnsson M., Samec J. S. M., Nat. Chem. 2021, 13, 1118-1125).

Although catalytic oxidative methods with the ability to cleave the C-C bonds of the alkyl side chains or even produce aromatic monomer products have been proposed, many are limited by poor selectivity and consequently low product yields (see e.g. Sedai B., Baker R. T., Adv. Synth. Catal. 2014, 356, 3563-3574; Wang M., Lu J., Li L., Li H., Liu H., Wang F., J. Catal. 2017, 348, 160-167; Schutyser W., Kruger J. S., Robinson A. M., Katahira R., Brandner D. G., Cleveland N. S., Mittal A., Peterson D. J., Meilan R., Román-Leshkov Y., Beckham G. T., Green Chem. 2018,20, 3828-3844; Liu M., Zhang Z., Shen X., Liu H., Zhang P., Chen B., Han B., Chem. Commun. 2019, 55, 925-928; Liu M., Zhang Z., Song J., Liu S., Liu H., Han B., Angew. Chem. Int. Ed. 2019, 58, 17393-17398; Liu J., Qiu X., Huang X., Luo X., Zhang C., Wei J., Pan J., Liang Y., Zhu Y., Qin Q., Song S., Jiao N., Nat. Chem. 2019,11, 71-77; Liu M., Zhang Z., Yan J., Liu S., Liu H., Liu Z., Wang W., He Z., Han B., Chem 2020, 6, 3288-3296; Luo H., Wang L., Shang S., Li G., Lv Y., Gao S., Dai W., Angew. Chem. Int. Ed. 2020, 59, 19268-19274; Zhou H., Li Z., Xu S.-M., Lu L., Xu M., Ji K., Ge R.,

Yan Y., Ma L., Kong X., Zheng L., Duan H., Angew. Chem. Int. Ed. 2021, 60, 8976-8982; Cui T., Ma L., Wang S., Ye C., Liang X., Zhang Z., Meng G., Zheng L., Hu H.-S., Zhang J., Duan H., Wang D., Li Y., J. Am. Chem. Soc. 2021, 143, 9429-9439; Ma L., Zhou H., Kong X., Li Z., Duan H., ACS Sustain. Chem. Eng. 2021, 9, 1932-1940; Shi S.-H., Liang Y., Jiao N., Chem. Rev. 2021, 121, 485-505.).

The critical issue that must be solved to overcome the aforementioned limitations is that the phenolic hydroxy group is unstable under oxidative conditions (see e.g. Rahimi A., Ulbrich A., Coon J. J., Stahl S. S., Nature 2014, 515, 249-252), leading to side reactions including repolymerization and ring opening reactions, which generates complex polymers, oligomers, and non-aromatic side products (see e.g. Esguerra K. V. N., Fall Y., Petitjean L., Lumb J.-P., J. Am. Chem. Soc. 2014,136, 7662-7668; Niederer K. A., Gilmartin P. H., Kozlowski M. C., ACS Catal. 2020, 10, 14615-14623; Xu W., Huang Z., Ji X., Lumb J.-P., ACS Catal. 2019, 9, 3800-3810; Neuhaus W. C., Kozlowski M. C., Angew. Chem. Int. Ed. 2020, 59, 7842-7847.). Hence, aromatic products are isolated in yields of < 5% in certain direct oxidative degradation reactions.

Proceeding from the prior art elucidated hereinabove, it is an object of the invention to provide a method for the transformation of lignin into aromatic compounds in good yields. The aromatic compounds can then be used as starting material for aromatic products. Another object of the invention was to provide a diaryl ether compound that can be manufactured from renewable resources.

Other and more specific objects will in part be apparent and will in part appear hereinafter.

SUMMARY OF THE INVENTION

Some or all of these objects are achieved according to the invention by the compound according to claim 1, the method according to claim 6, and the use according to claim 14.

Further advantageous embodiments of the invention are specified in the dependent claims and are elucidated in detail herein below.

It was surpriginsly found that with the present invention, the drawbacks frequently encountered in the methods known in the prior art could be at least partially overcome. In particular, it was found that the present invention provides an efficient route to extract the aromatic monomers from lignin affording functionalized diaryl ethers. Moreover, the invention can overcome the limitations of oxidative methods described in the prior art and can afford high-value specialty chemicals in good yields. Further, side reactions typically encountered in oxidative depolymerization reactions of lignin may be suppressed with the present invention.

Without wishing to be bound by scientific theory, it is believed that in the method according to the invention, the hydroxyl groups in the repeat units of lignin depicted above are oxidized followed by cleavage of the bond thereinbetween (the bond between the α-carbon and the β-carbon, relative to the aromatic ring). This is followed by further bond cleavage to yield the free phenolic oxygen that subsequently reacts with the aryl boronic compound having the structure (II) (see below).

DESCRIPTION OF PREFERRED EMBODIMENTS

Compound according to the reaction

The invention provides for a compound having the structure (I)

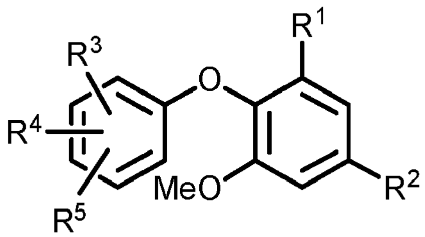

(I),

wherein

$R^1$ is H or -OMe,

$R^2$ is -CHO, $-CO_2H$, $C_1$-$C_4$-alkoxycarbonyl, or $C_3$-$C_6$-cycloalkoxycarbonyl,

$R^3$, $R^4$, and $R^5$ are independently of each other H, halogen, halogenated $C_1$-$C_4$-alkyl, Cs-$C_{10}$-aryl, in particular

phenyl, nitro, $C_1$-$C_4$-alkoxy, $C_1$-$C_4$-alkoxycarbonyl, or $C_3$-$C_6$- cycloalkoxycarbonyl.

In the compound having the structure (I), $R^2$ is preferably-CHO, -$CO_2H$, or -$CO_2Me$, more preferably -CHO or -$CO_2H$, most preferred -$CO_2H$. These compounds are valuable intermediates for other compounds.

In the compound having the structure (I), the substituents $R^3$, $R^4$, and $R^5$ are preferably independently of each other H, F, Cl, Br, perfluorinated $C_1$-$C_4$-alkyl, preferably -$CF_3$, -$NO_2$, -OMe, or -$CO_2Me$. The aforementioned substituents provide access to a range of intermediates that may be relevant for example for pharmaceutical compounds.

According to an embodiment, $R^3$ is as defined herein and $R^4$ and $R^5$ are H.

In the compound having the structure (I), the substituents $R^3$, $R^4$, and $R^5$ may be freely positioned on the aromatic ring, wherein only one substituent may be present per carbon atom in the aromatic ring. Preferably, $R^3$ is in para position.

According to a preferred embodiment, the compound according to the invention has the structure (Ia):

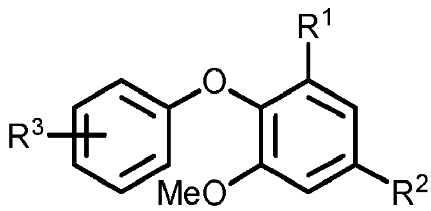

(Ia),

wherein $R^1$, $R^2$, and $R^3$ are as defined herein. Preferably, the compound has the structure (Ia), wherein

$R^1$ is H or -OMe,

$R^2$ is -CHO or -$CO_2H$, and

$R^3$ is H, F, Cl, Br,-$CF_3$, -$NO_2$, -OMe, or -$CO_2Me$, preferably wherein $R^3$ is in para position.

According to a preferred embodiment, the compound according to the invention has one of the following structures:

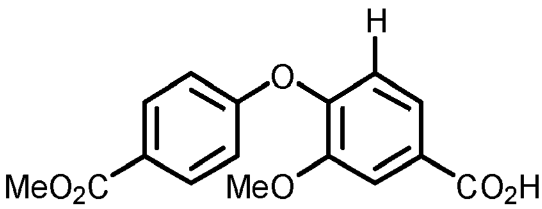, 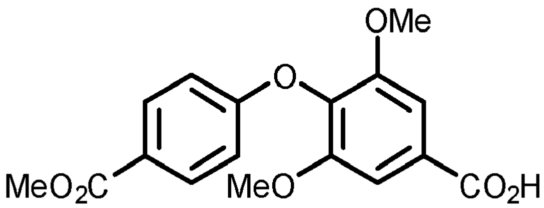,

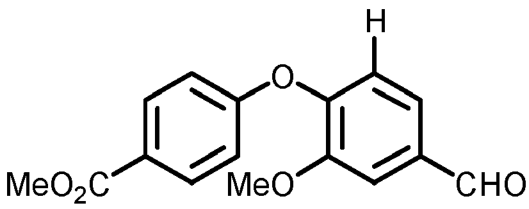, 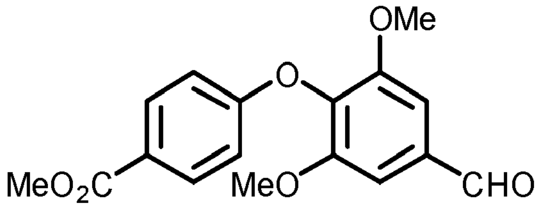,

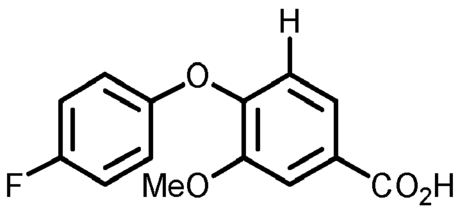, 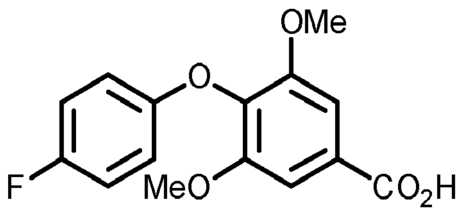,

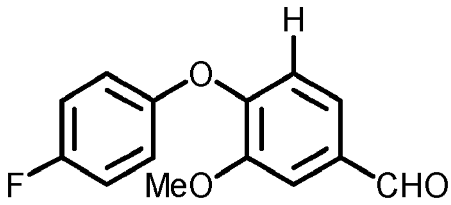,
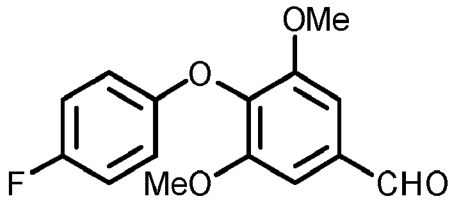,

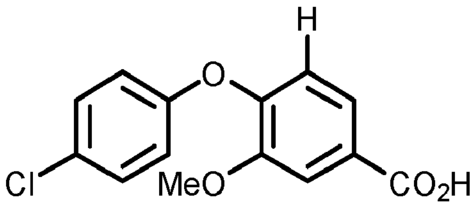,
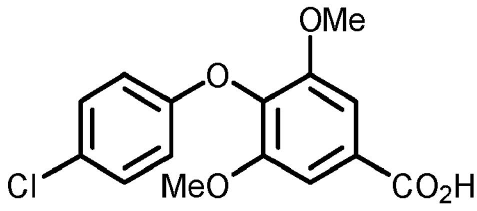,

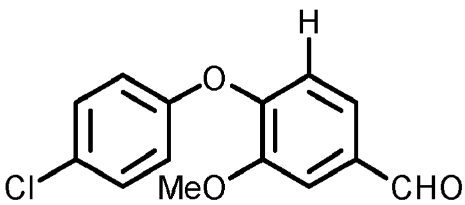,
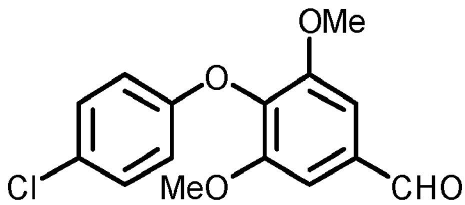,

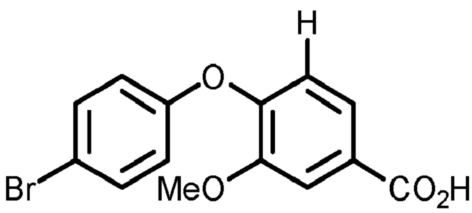,
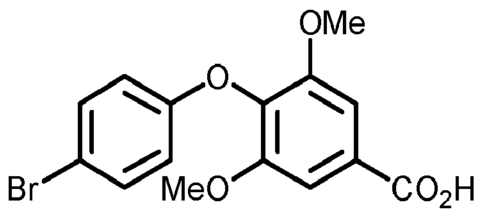,

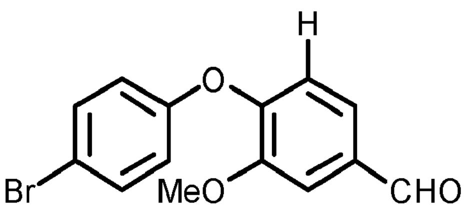,
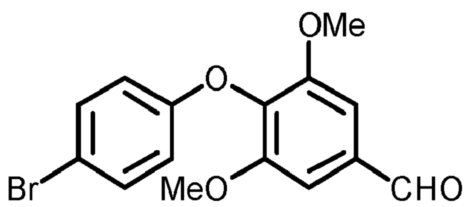,

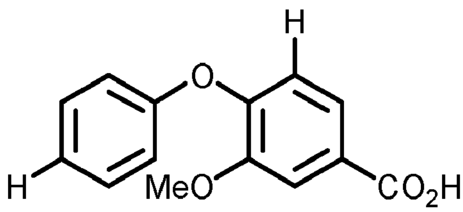,
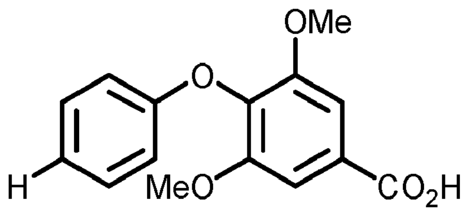,

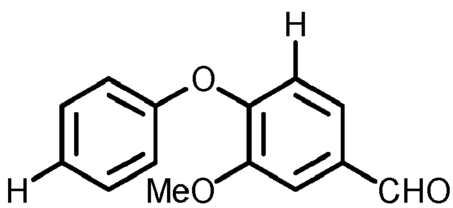,
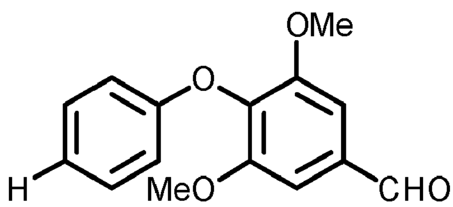,

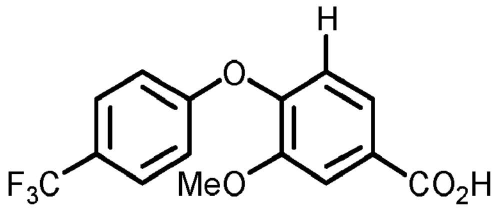, 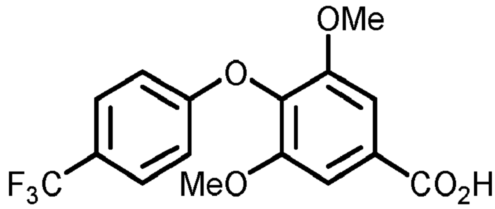,

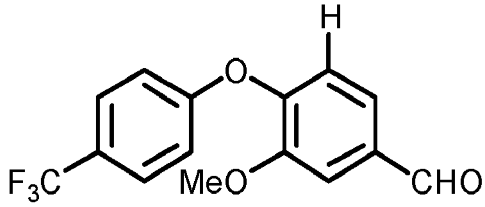, 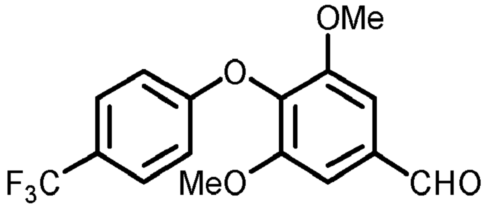,

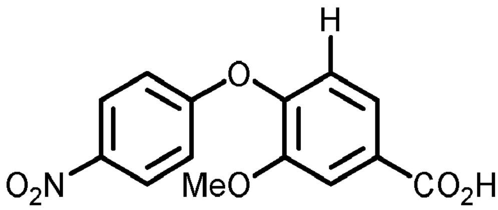, 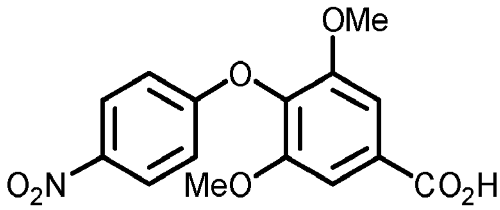,

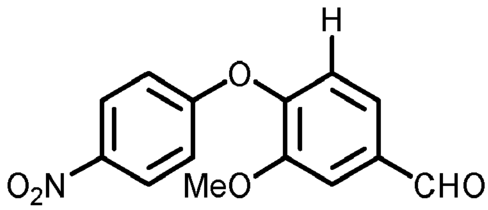, 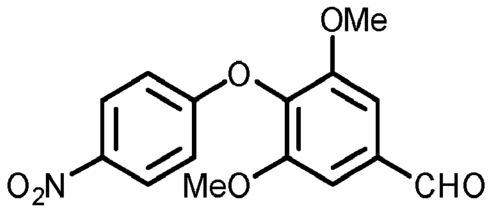,

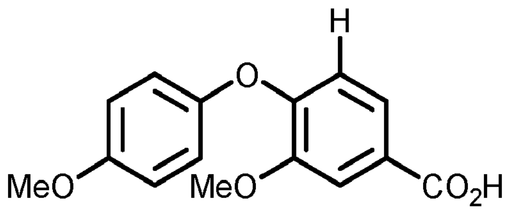, 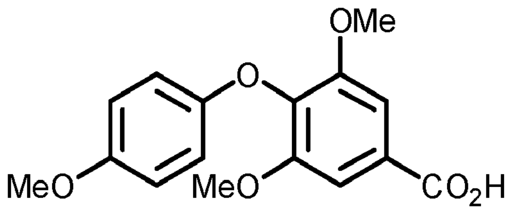,

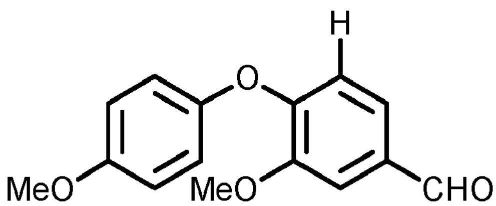, or 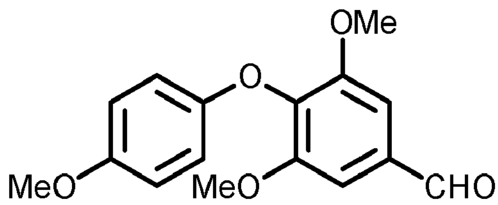.

Method according to the invention

The invention also provides for a method for the manufacture of a diaryl ether compound described herein, wherein lignin is reacted with an aryl boronic compound having the structure (II)

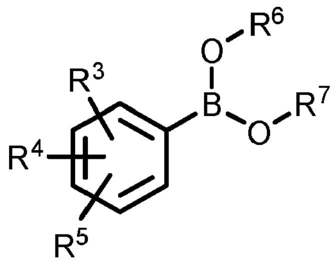

(II),

wherein

$R^3$, $R^4$, and $R^5$ are as defined herein above,

$R^6$ and $R^7$ are independently of each other H, $C_1$-$C_4$-alkyl, or $R^6$ and $R^7$ together with the oxygen atoms and the boron atom form a 5- or 6-membered ring optionally substituted with 1 to 4 $C_1$-$C_3$-alkyl substituents.

According to an embodiment, $R^6$ and $R^7$ are H, or $R^6$ and $R^7$ form together with the oxygen atoms and the boron atom a pinakolboryl residue or a neopentylglycolboryl residue. Hence, in the aryl boronic compound having the structure (II), $R^6$ and $R^7$ may preferably be H or the aryl boronic compound may have the structure (IIa) or (IIb)

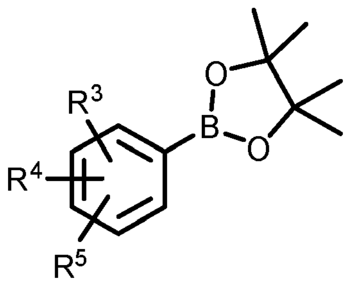

(IIa) or

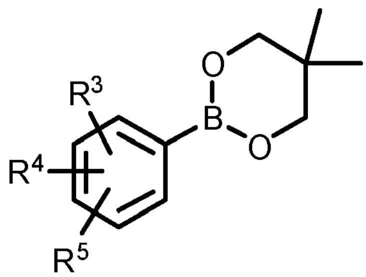

(IIb).

The lignin used in the method according to the invention may be from different sources. Preferably the lignin comes from grass and/or wood, in particular softwood and/or hardwood. Examples for wood are beech wood, poplar wood, pine wood, and oak wood. Advantageously, the lignin may be beech lignin, poplar lignin, pine lignin, and/or oak lignin. Preferably, lignin contains the following repeat units

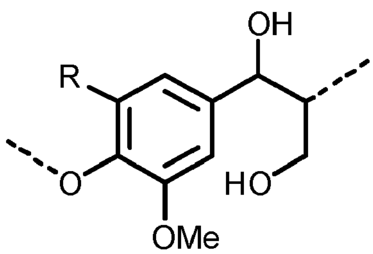

R = H or OMe .

Lignin preferably contains β-O-4 linkages. In the repeat units depicted above, a β-O-4 linkage is present from the carbon atom in β-position from the aryl ring to the oxygen atom of an adjacent repeat unit, depicted as a dashed bond (oxygen atom of the adjacent repeat unit not shown). The amount of β-O-4 linkages in lignin can be determined for example using NMR spectroscopy, in particular using HSQC NMR spectroscopy. The amount of β-O-4 linkages in lignin refers to the molar amount of β-O-4 linkages in lignin.

In the method according to the invention, beech lignin is preferably employed.

According to an embodiment, the lignin is extracted from grass or wood. Extraction of the lignin may be accomplished by mixing the grass or wood with an organic solvent, for example tetrahydrofuran or dioxane, and an aqueous

acidic solution, preferably in an extraction vessel. The mixture may be heated to 90 to 130°C, preferably to 100 to 120°C. The heating may be conducted for 0.5 to 2 hours. The reaction mixture may then be filtered and washed with dioxane and concentrated to obtain a concentrated mixture. The concentrated mixture may then be dissolved in acetone and water (v/v 9:1) and the solution may be precipitated in water. The precipitate may be collected and dried to yield crude lignin. The crude lignin may be dissolved in acetone and methanol (v/v 9:1) and precipitated in diethyl ether, filtered and dried under vacuum to yield lignin.

In the method according to the invention, the lignin is preferably reacted with the aryl boronic compound having the structure (II) in the presence of a transition metal salt.

Various transition metal salts may be used in the method according to the invention. Preferably the transition metal salt is a copper salt, a palladium salt, or a ruthenium salt, even more preferably a copper salt. Examples for suitable copper salts are copper oxide, copper sulfate, copper(I) chloride, copper(II) chloride, copper nitrate, $Cu(BF_4)_2$, copper acetate, copper perchlorate, copper(II) bis(2-ethylhexanoate), copper(II) 2-thiophenecarboxylate, copper(II) 2-pyrazinecarboxylate, copper bis(acetylacetonate), and copper triflate, preferably copper sulfate, copper(I) chloride, copper(II) chloride, copper nitrate, $Cu(BF_4)_2$, copper acetate, copper perchlorate, copper(II) bis(2-ethylhexanoate), copper(II) 2-thiophenecarboxylate, copper(II) 2-pyrazinecarboxylate, copper bis(acetylacetonate), and copper triflate, more preferably copper(I) chloride, copper(II) chloride, copper nitrate, $Cu(BF_4)_2$, copper acetate, copper perchlorate, copper(II) bis(2-ethylhexanoate), copper(II) 2-thiophenecarboxylate, copper(II) 2-pyrazinecarboxylate, and copper triflate, even more preferably copper(I) chloride, copper nitrate, and copper triflate. Most preferably, the lignin is reacted with the aryl boronic compound having the structure (II) in the presence of copper triflate.

The transition metal salt is advantageously present in the method according to the invention in an amount of at least 1 mol%, preferably at least 5 mol%, more preferably at least 10 mol%, based on the total amount of β-O-4 linkages in the lignin. The upper limit for the amount of transition metal salt in the method according to the invention is preferably 50 mol% or less, more preferably 40 mol% or less, based on the total amount of β-O-4 linkages in the lignin. According to an embodiment of the invention, the transition metal salt is present in an amount of 5 to 40 mol%, preferably 5 to 35 mol%, more preferably 10 to 35 mol%, based on the total amount of β-O-4 linkages in the lignin.

According to an embodiment of the invention, the lignin is reacted with the aryl boronic compound having the structure (II) in the presence of a ligand. Different ligands can be used. Advantageously, the ligand is a nitrogen-containing or a phosphorous-containing aromatic or non-aromatic organic compound, in particular monodentate or bidentate aliphatic or aromatic nitrogen-donor-ligands or monodentate or bidentate aliphatic or aromatic phosphorous-donor-ligands, such as a $C_1$-$C_{10}$-alkylamine, a $C_2$-$C_{10}$-alkylenediamine, a pyridine compound, a biphenyl compound or a phenanthroline compound in each case optionally substituted with one to four $C_1$-$C_4$-alkyl substituents, one to four $C_1$-$C_4$-alkoxy substituents, one to four phenyl substituents, one to four hydroxyl groups, one to four oligoethylene glycol groups, or one to four halogen atoms, in particular chlorine atoms, or such as a tri-($C_1$-$C_4$-alkyl)phosphine, a bis-[di-($C_1$-$C_4$-alkyl)] phosphine bridged by an $C_1$-$C_4$-alkylene bridge or a tri-($C_6$-$C_{10}$-aryl)phosphine or a bis-[di-($C_6$-$C_{10}$-aryl)]phosphine bridged by an $C_1$-$C_4$-alkylene bridge. Preferably, the lignin is reacted with the aryl boronic compound having the structure (II) in the presence of a nitrogen-containing aromatic ligand, more preferably a nitrogen-containing aromatic ligand selected from the group consisting of

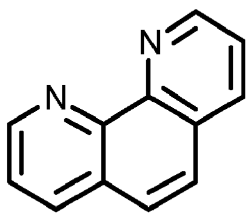

(L-I),

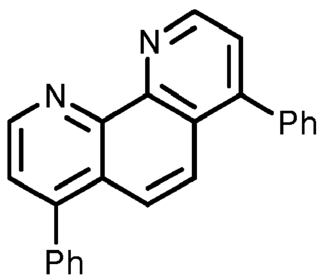

(L-II),

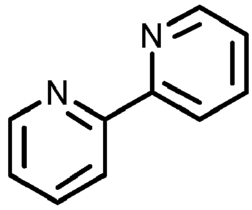

(L-III),

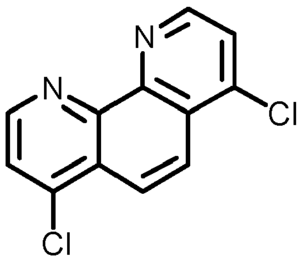

(L-IV), and

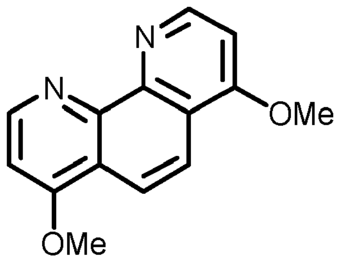

(L-V),

even more preferably a nitrogen-containing aromatic ligand selected from the group consisting of

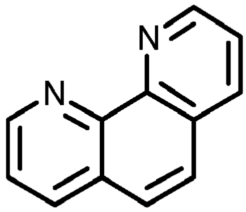

(L-I),

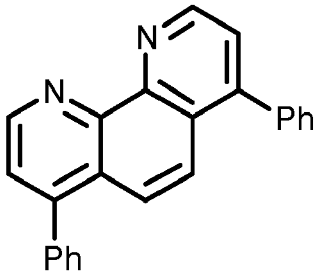

(L-II), and

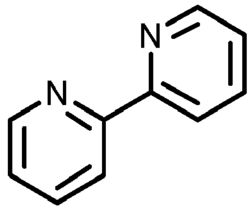

(L-III).

Most preferably, the lignin is reacted with the aryl boronic compound having the structure (II) in the presence of

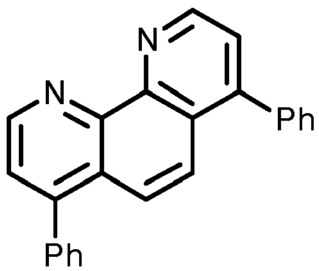

(L-II) as ligand.

The ligand, in particular the nitrogen-containing aromatic ligand, is advantageously present in the method according to the invention in an amount of at least 1 mol%, preferably at least 5 mol%, more preferably at least 10 mol%, based on the total amount of β-O-4 linkages in the lignin. The upper limit for the amount of the ligand, in particular the nitrogen-containing aromatic ligand, in the method according to the invention is preferably 50 mol% or less, more preferably 40 mol% or less, based on the total amount of β-O-4 linkages in the lignin. According to an embodiment of the invention, the ligand, in particular the nitrogen-containing aromatic ligand, is present in an amount of 5 to 40 mol%, preferably 5 to 35 mol%, more preferably 10 to 35 mol%, based on the total amount of β-O-4 linkages in the lignin.

In the method according to the invention, the lignin is preferably reacted with the aryl boronic compound having the structure (II) under oxidizing conditions. Oxidizing conditions can be achieved in various ways. Preferably, the lignin is reacted in the presence of an oxidizing agent such as peroxides, permanganates, chrome-based oxidizing agents, $BrO_3^-$, ClO-, air, or oxygen. Most preferred, the lignin is reacted in the presence of oxygen. Oxygen is preferably used at a pressure of 0.5 to 10 atmospheres, more preferably 0.5 to 8 atmospheres, most preferred 1 to 5 atmospheres.

According to a preferred embodiment, the lignin is reacted with the aryl boronic compound having the structure (II) in the presence of a transition metal salt, in particular as described herein, and a nitrogen-containing aromatic ligand, in particular as described herein, preferably under oxidizing conditions, in particular as described herein.

In the method according to the invention, the lignin is preferably reacted with the aryl boronic compound having the structure (II) in the presence of a base. The base may be an organic base or an inorganic base, preferably an inorganic base. Examples of suitable inorganic bases are alkaline metal hydroxides or alkaline metal carbonates or alkaline earth metal hydroxides or alkaline earth metal carbonates, preferably alkaline metal carbonates or hydroxides,

more preferably NaOH, KOH, $Na_2CO_3$, $K_2CO_3$, and/or $Cs_2CO_3$, most preferred $K_2CO_3$.

If a base is used, the base may be present in various amounts. Preferably, the lignin is reacted with the aryl boronic compound having the structure (II) in the presence of a base in the amount of 1.0 to 10.0 equivalents, preferably from 2.0 to 8.0 equivalents, more preferably 2.0 to 6.0 equivalents, most preferred from 3.0 to 5.0 equivalents, based on the total amount of β-O-4 linkages in the lignin.

According to another preferred embodiment, the lignin is reacted with the aryl boronic compound having the structure (II) in the presence of a transition metal salt, in particular as described herein, a base, in particular as described herein, and a nitrogen-containing aromatic ligand, in particular as described herein, preferably under oxidizing conditions, in particular as described herein.

For reacting the lignin with the aryl boronic compound having the structure (II) in the method according to the invention, a solvent is advantageously used. Various solvents can be used in the method according to the invention. The solvent is advantageously an organic solvent, preferably a dipolar aprotic organic solvent or water. More preferably, the lignin is reacted with the aryl boronic compound having the structure (II) in the presence of a solvent selected from the group consisting of dimethylformamide, dimethylacetamide, N-methylpyrrolidone, dimethylsulfoxide, acetonitrile, water, methanol, dioxane, dichloromethane, and mixtures thereof, even more preferably in the presence of a solvent selected from the group consisting of dimethylformamide, dimethylacetamide, N-methylpyrrolidone, dimethylsulfoxide, acetonitrile, water, and mixtures thereof, most preferred in the presence of a solvent selected from the group consisting of dimethylformamide, dimethylacetamide, N-methylpyrrolidone, dimethylsulfoxide, acetonitrile, and mixtures thereof. According to a preferred embodiment, the solvent is dimethylsulfoxide. These solvents provide sufficient solubility for the compounds in the reaction.

In the method according to the invention, the lignin is preferably reacted with the aryl boronic compound having the structure (II) for a duration of 1 to 12 hours, more preferably 1 to 10 hours, even more preferably 2 to 9 hours, most preferably 4 to 8 hours.

The method according to the invention can be conducted at various temperatures. Advantageously, the lignin is reacted with the aryl boronic compound having the structure (II) at a temperature from 80 to 200°C, preferably from 100 to 180°C, more preferably from 120 to 160°C, most preferably from 130 to 150°C. At these temperatures, the reaction proceeds sufficiently fast while the compounds are not degraded.

In the method according to the invention, the aryl boronic compound having the structure (II) may be used in different amounts. Advantageously, the aryl boronic compound having the structure (II) is employed in an amount of 1.0 to 3.0 equivalents, preferably 1.0 to 2.5 equivalents, more preferably 1.0 to 2.0 equivalents, based on the total amount of β-O-4 linkages in the lignin, in the method according to the invention. With the aryl boronic compound in these amounts, a high conversion of the lignin is achieved while the amount of chemical waste is still limited.

With the method according to the invention, different diaryl ether compounds having the structure (I)

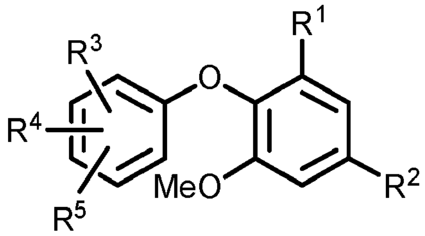

(I)

may be obtained as described above. For example, with the method according to the invention, $R^2$ in the diaryl ether compound may be -CHO or $-CO_2H$.

The method according to the invention may include further steps. For example, the method according to the invention may contain an esterification step, for example methylation, butylation or benzylation. An esterification step, in particular a methylation, is preferably conducted at 10 to 100 °C, more preferably from 15 to 50°C. An esterification step, in particular a methylation, is preferably conducted for 8 to 16 hours, more preferably for 10 to 14 hours.

The method according to the invention may also contain a work-up step. In particular, after reacting the lignin with the aryl boronic compound having the structure (II), the resulting mixture may be treated with ethyl acetate and a saturated ammonium chloride solution to obtain an extract of the organic products. The extract of the organic products may be filtered over silica gel to remove inorganic compounds as well as any ligands employed.

The invention also relates to the use of an aryl boronic compound having the structure (II)

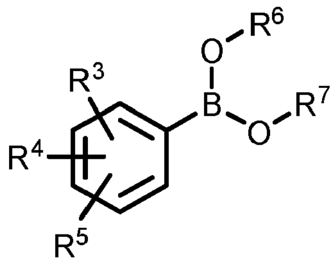

(II)

in the conversion of lignin to a diaryl ether compound,
wherein $R^3$, $R^4$, $R^5$, $R^6$, $R^7$ are as defined herein.

Fig. 1a to 1c shows HSQC NMR spectra of mixtures of trioxane and 1-(3,4-dimethoxyphenyl)-2-(2-methoxyphenoxy)propane-1,3-diol at different concentrations including a coarse peak assignment and the integrals of the respective peaks,

Fig. 2 shows an HSQC NMR spectrum of isolated beech wood lignin before the reaction. The insert table summaries the occurrence values of bonding motifs and potential linkages of lignin (mmol per 1 g lignin). Trioxane was used as an internal standard to calculate the content of the corresponding structures. For this spectrum, 54.6 mg beech lignin and 4.5 mg trioxane was dissolved in 0.5 mL DMSO-$d_6$. Also shown are the typical structural elements in beech lignin and the assignments of particular protons of these structures in the HSQC NMR spectrum.

Fig. 3 $^1$H NMR spectrum in $CDCl_3$ of

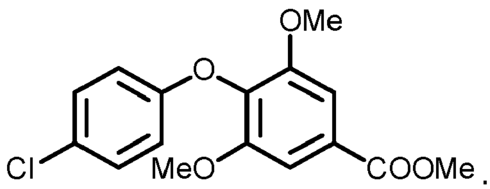

Fig. 4 $^{13}$C NMR spectrum in $CDCl_3$ of

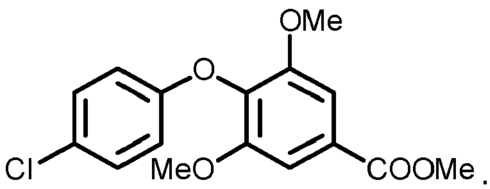

Fig. 5 $^1$H NMR spectrum in $CDCl_3$ of

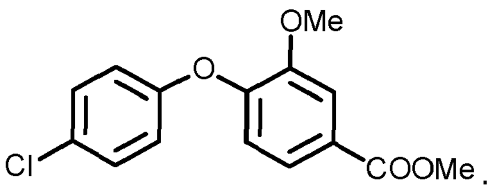

Fig. 6 $^{13}$C NMR spectrum in $CDCl_3$ of

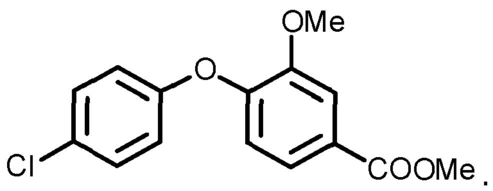

Fig. 7 $^{1}H$ NMR spectrum in $CDCl_3$ of

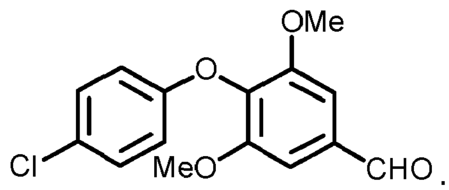

Fig. 8 $^{13}C$ NMR spectrum in $CDCl_3$ of

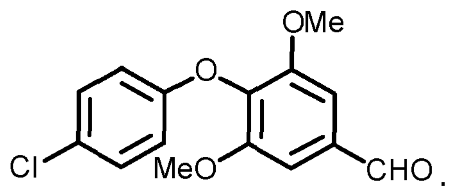

Fig. 9 $^{1}H$ NMR spectrum in $CDCl_3$ of

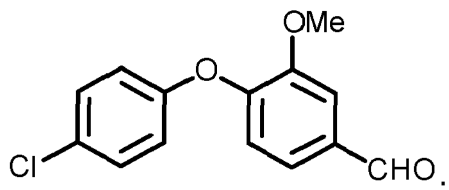

Fig. 10 $^{13}C$ NMR spectrum in $CDCl_3$ of

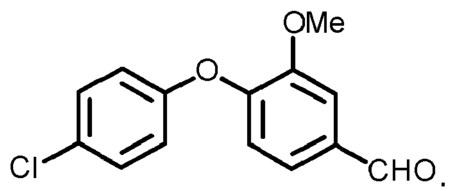

Fig. 11 $^{1}H$ NMR spectrum in $CDCl_3$ of

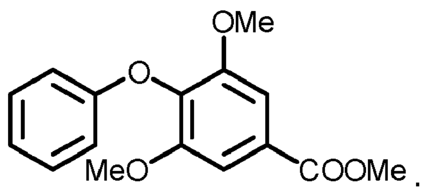

Fig. 12 $^{13}C$ NMR spectrum in $CDCl_3$ of

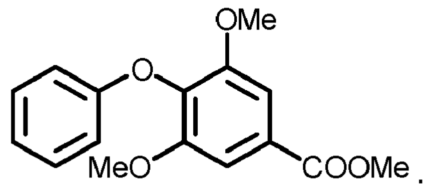

Fig. 13 $^{1}H$ NMR spectrum in $CDCl_3$ of

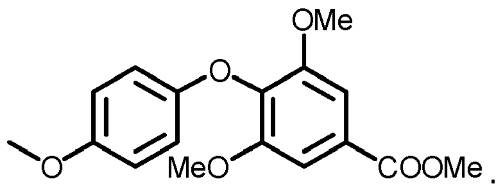

Fig. 14 $^{13}C$ NMR spectrum in $CDCl_3$ of

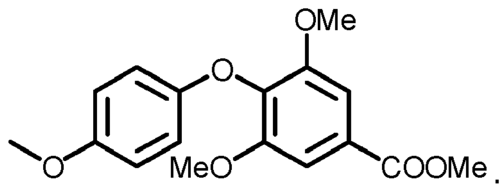

Fig. 15 $^{1}H$ NMR spectrum in $CDCl_3$ of

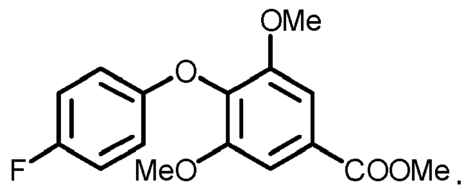

Fig. 16 $^{13}C$ NMR spectrum in $CDCl_3$ of

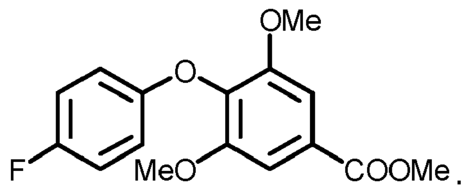

Fig. 17 $^{1}H$ NMR spectrum in $CDCl_3$ of

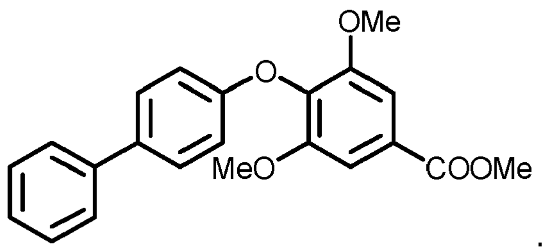

Fig. 18 $^{13}C$ NMR spectrum in $CDCl_3$ of

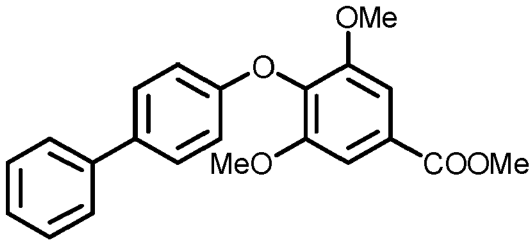
.

Fig. 19 $^{1}H$ NMR spectrum in $CDCl_3$ of

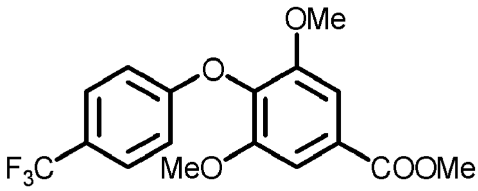
.

Fig. 20 $^{13}C$ NMR spectrum in $CDCl_3$ of

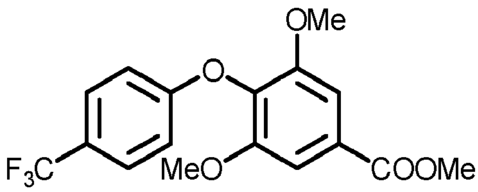
.

Fig. 21 $^{1}H$ NMR spectrum in $CDCl_3$ of

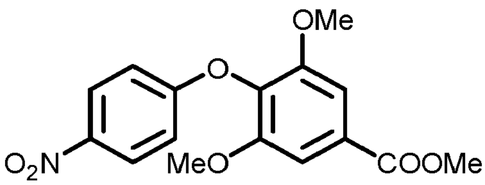
.

Fig. 22 $^{13}C$ NMR spectrum in $CDCl_3$ of

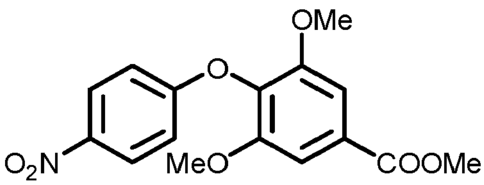
.

Fig. 23 $^{1}H$ NMR spectrum in $CDCl_3$ of

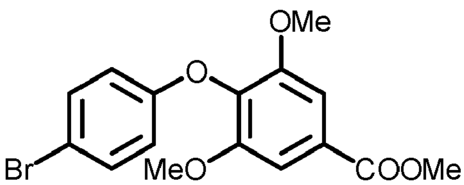
.

Fig. 24 $^{13}C$ NMR spectrum in $CDCl_3$ of

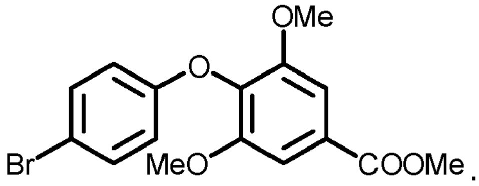

Fig. 25 $^{1}H$ NMR spectrum in $CDCl_3$ of

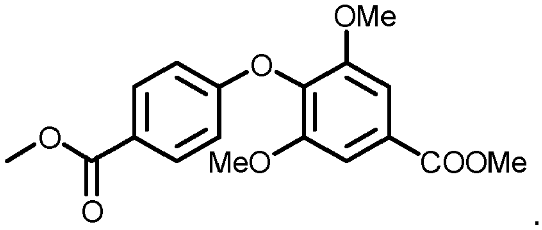

Fig. 26 $^{13}C$ NMR spectrum in $CDCl_3$ of

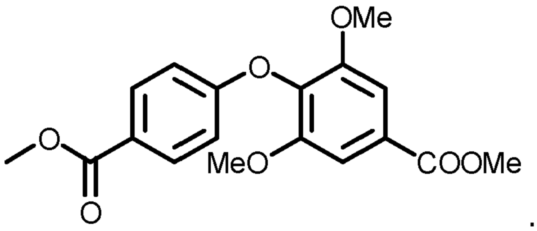

Fig. 27 gas chromatogram of the reaction mixture to yield the main product methyl 4-(4-chlorophenoxy)-3,5-dimethoxybenzoate from beech lignin.

## EXAMPLES

### Materials and methods

#### Materials

Commercially available Cu salt catalysts were purchased from Alfa Aesar and cocatalysts (ligands) were purchased from Strem Chemicals. Bases, boronic acids, and other reagents were obtained from Sigma-Aldrich. Solvents were purchased from Acros Organics. Compressed air and oxygen were purchased from Carbagas.

#### Lignin extraction

Wood sawdust (< 0.5 mm) was dried at 80 °C for 24 h prior to lignin extraction. Wood sawdust (50 g), dioxane (360 mL), and HCl aqueous solution (40 mL, 2 mol/L) were added to a flask and protected under $N_2$. The mixture was heated to reflux for 1 h (~ 110 ºC). The mixture was filtered, washed with dioxane and concentrated to afford a brown liquid-gel (~ 100 mL). The liquid-gel was dissolved in a mixture of acetone and $H_2O$ (v/v = 9:1, 250 mL). Crude lignin was obtained by the precipitation of the solution in $H_2O$ (2 L) and dried using a lyophilizer. Crude lignin was dissolved in the mixture of acetone and methanol (v/v = 9:1, 200 mL, if not fully dissolved, further methanol was added). The lignin was regenerated by precipitation in diethyl ether (2 L), filtered and dried under vacuum.

#### Exemplary reaction to prepare diaryl ether compounds from lignin

Beech lignin (40 mg, content of β-O-4 linkages is 0.245 mmol/g lignin), 4-chlorophenylboronic acid (1.5 equiv.), $Cu(OTf)_2$ (30 mol%), bathophenanthroline (Bphen, 30 mol%), $K_2CO_3$ (4 equiv.), biphenyl (as internal standard for gas chromatography, 2 equiv.), and DMSO (2 mL) were added into an autoclave. The reactor was heated to 140 °C for 6 h. After cooling to room temperature, the crude product was methylated in a second step by addition of $K_2CO_3$ (3 equiv.)

and MeI (10 equiv.) followed by stirring for 12 h. After the reaction, ethyl acetate (EA, 3 mL) and a saturated $NH_4Cl$ (5 mL) aqueous solution were added to the mixture to extract the organic products. The aqueous solution was extracted with EA (3x3 mL). For qualitative and quantitative analysis of the products, the combined organic phase was filtered over silica gel to remove the inorganic salts and ligand. Further purification was achieved using thin layer chromatography with hexane: ethyl acetate (0 to 10%) to yield methyl 4-(4-chlorophenoxy)-3,5-dimethoxybenzoate as a white powder or crystal (1.9 mg, 60% isolated yield). A gas chromatogram of the reaction mixture is shown in Figure 27, wherein the assignment of the main species is also shown in Figure 27.

According to the exemplary method described above, several syntheses as compiled in Table 1 were performed using different copper salts, ligands, bases, and solvents, and the yields as reported in Table 1 were obtained in these syntheses.

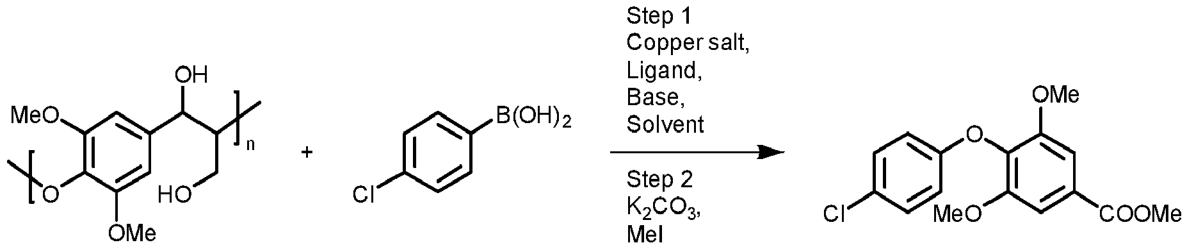


With the reaction above, a mixture of the diaryl ethers

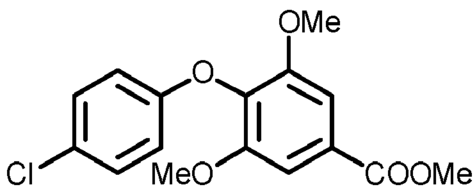
,
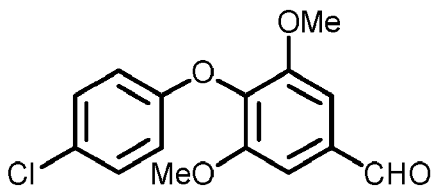
,

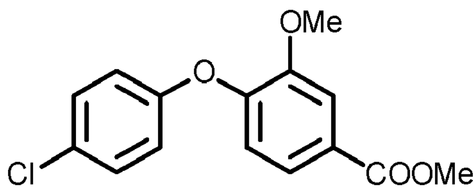
, and
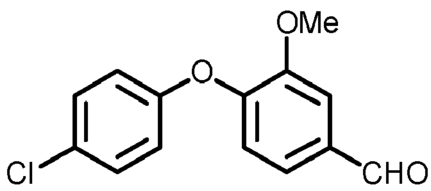

was obtained with methyl 4-(4-chlorophenoxy)-3,5-dimethoxybenzoate being the major product $^1H$ and $^{13}C$ NMR spectra of these compounds are shown in Figures 3 to 10. The combined yields of these diaryl ether products are reported in Table 1 as the yield of all diaryl ether products.

Table 1: Syntheses performed and respective yields

| Exp No. | Copper Salt | Ligand | Base | Solvent | Yield[a] | Yield[b] |
|---|---|---|---|---|---|---|
| 1 | $Cu(NO_3)_2$*$3H_2O$ | L-II | $K_2CO_3$ | DMSO | 57 | 83 |
| 2 | Cu(BF4)2 | L-II | $K_2CO_3$ | DMSO | 53 | 79 |
| 3 | $Cu(OTf)_2$ | L-II | $K_2CO_3$ | DMSO | 68 | 92 |
| 4 | CuCl | L-II | $K_2CO_3$ | DMSO | 42 | 54 |
| 5 | $CuCl_2$ | L-II | $K_2CO_3$ | DMSO | 36 | 55 |
| 6 | $Cu(OAc)_2$ | L-II | $K_2CO_3$ | DMSO | 34 | 51 |
| 7 | $Cu(ClO_4)_2$ | L-II | $K_2CO_3$ | DMSO | 44 | 63 |
| 8 | Cu(acac)2 | L-II | $K_2CO_3$ | DMSO | 20 | 28 |
| 9 | Cu(II) bis(2-ethylhexanoate ) | L-II | $K_2CO_3$ | DMSO | 30 | 48 |

(continued)

| Exp No. | Copper Salt | Ligand | Base | Solvent | Yield[a] | Yield[b] |
|---|---|---|---|---|---|---|
| 10 | Cu(II) 2-thio-phenecarbo-xylate | L-II | $K_2CO_3$ | DMSO | 45 | 67 |
| 11 | Cu(II) 2-pyra-zinecarbo-xylate | L-II | $K_2CO_3$ | DMSO | 47 | 68 |
| 12 | $Cu(OTf)_2$ | L-II | $Cs_2CO_3$ | DMSO | 59 | 81 |
| 13 | $Cu(OTf)_2$ | L-II | $Na_2CO_3$ | DMSO | 50 | 83 |
| 14 | $Cu(OTf)_2$ | L-II | $Li_2CO_3$ | DMSO | 37 | 69 |
| 15 | $Cu(OTf)_2$ | L-II | NaOH | DMSO | 61 | 93 |
| 16 | $Cu(OTf)_2$ | L-II | KOH | DMSO | 55 | 90 |
| 17 | $Cu(OTf)_2$ | L-II | $K_3PO_4$ | DMSO | 40 | 71 |
| 18 | $Cu(OTf)_2$ | L-II | $KOC(CH_3)_3$ | DMSO | 34 | 47 |
| 19 | $Cu(OTf)_2$ | L-II | $NaOCH_2CH_3$ | DMSO | 40 | 71 |
| 20 | $Cu(OTf)_2$ | L-II | $NaOCH_3$ | DMSO | 42 | 76 |
| 21 | $Cu(OTf)_2$ | L-I | $K_2CO_3$ | DMSO | 51 | 72 |
| 22 | $Cu(OTf)_2$ | L-III | $K_2CO_3$ | DMSO | 37 | 46 |
| 23 | $Cu(OTf)_2$ | L-IV | $K_2CO_3$ | DMSO | 53 | 79 |
| 24 | $Cu(OTf)_2$ | L-V | $K_2CO_3$ | DMSO | 57 | 74 |
| 25 | $Cu(OTf)_2$ | L-VI | $K_2CO_3$ | DMSO | 11 | 19 |
| 26 | $Cu(OTf)_2$ | pyridine | $K_2CO_3$ | DMSO | 10 | 14 |
| 27 | $Cu(OTf)_2$ | L-II | $K_2CO_3$ | Water | 0 | 0 |
| 28 | $Cu(OTf)_2$ | L-II | $K_2CO_3$ | DMF | 35 | 52 |
| 29 | $Cu(OTf)_2$ | L-II | $K_2CO_3$ | NMP | 28 | 47 |
| 30 | $Cu(OTf)_2$ | L-II | $K_2CO_3$ | $CH_3CN$ | 11 | 19 |
| 31 | $Cu(OTf)_2$ | L-II | $K_2CO_3$ | Dioxan e | 0 | 0 |
| 32 | $Cu(OTf)_2$ | L-II | $K_2CO_3$ | $CH_2Cl_2$ | 0 | 0 |

Explanations to Table 1: [a] - yield of methyl 4-(4-chlorophenoxy)-3,5-dimethoxybenzoate obtained in %, determined by GC (see below), based on the amount of β-O-4 linkages in the lignin (see below); [b] - yield of all diaryl ether products obtained in %, determined by GC (see below) based on the amount of β-O-4 linkages in the lignin (see below).

Table 2: Structures and abbreviations of the ligands listed in Table 1.

| | | |
|---|---|---|
| 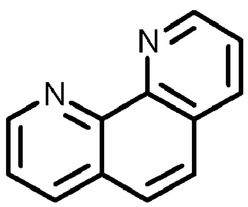 | 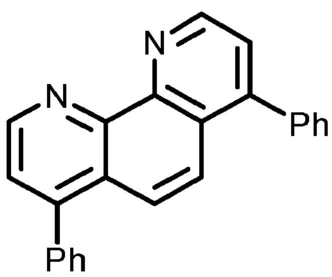 | 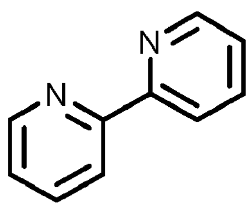 |
| L-I | L-II | L-III |

(continued)

| | | |
|---|---|---|
| 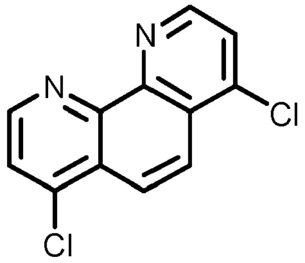 | 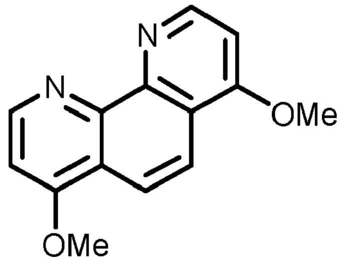 | 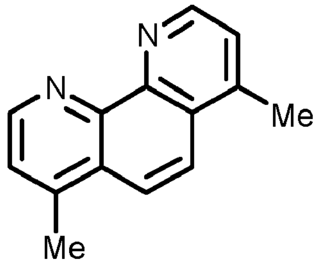 |
| L-IV | L-V | L-VI |

According to the exemplary method above, several syntheses as compiled in Table 3 were performed using different amounts of $Cu(OTf)_2$, of the bathophenanthroline ligand, of the amount of potassium carbonate base, of the atmosphere, of the reaction time, and of the reaction temperature, and the yields as reported in Table 3 were obtained.

Using the reaction, a mixture of the diaryl ethers

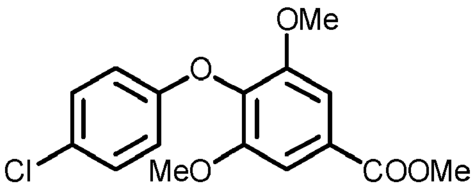, 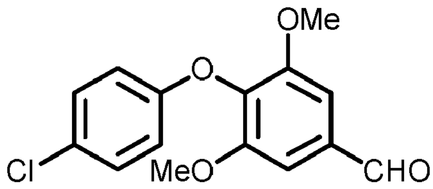,

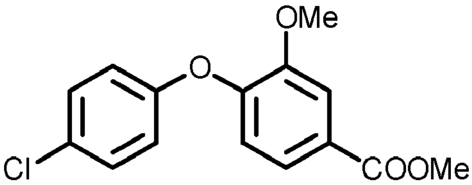, and 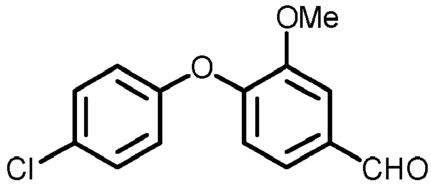

was obtained with methyl 4-(4-chlorophenoxy)-3,5-dimethoxybenzoate being the major product. The combined yields of these diaryl ether products are reported in Table 3 as the yield of all diaryl ether products.

Table 3: Syntheses performed and respective yields

| Exp No. | Cat[a] | Lig[b] | Base[c] | Atmos[d] | Duration[e] | Temp[f] | Yield[g] |
|---|---|---|---|---|---|---|---|
| 33 | 15 | 15 | 4 | $O_2$, 3 atm | 6 | 140 | 13 (15) |
| 34 | 30 | 30 | 4 | $O_2$, 3 atm | 6 | 140 | 68 (92) |
| 35 | 60 | 60 | 4 | $O_2$, 3 atm | 6 | 140 | 57 (84) |
| 36 | 100 | 100 | 4 | $O_2$, 3 atm | 6 | 140 | 44 (76) |
| 37 | 30 | 0 | 4 | $O_2$, 3 atm | 6 | 140 | 29 (41) |
| 38 | 30 | 15 | 4 | $O_2$, 3 atm | 6 | 140 | 55 (71) |
| 39 | 30 | 80 | 4 | $O_2$, 3 atm | 6 | 140 | 42 (65) |
| 40 | 30 | 30 | 1 | $O_2$, 3 atm | 6 | 140 | 28 (51) |
| 41 | 30 | 30 | 2 | $O_2$, 3 atm | 6 | 140 | 41 (59) |
| 42 | 30 | 30 | 3 | $O_2$, 3 atm | 6 | 140 | 55 (77) |
| 43 | 30 | 30 | 5 | $O_2$, 3 atm | 6 | 140 | 60 (83) |
| 44 | 30 | 30 | 6 | $O_2$, 3 atm | 6 | 140 | 33 (54) |

(continued)

| Exp No. | Cat[a] | Lig[b] | Base[c] | Atmos[d] | Duration[e] | Temp[f] | Yield[g] |
|---|---|---|---|---|---|---|---|
| 45 | 30 | 30 | 4 | $O_2$, 1 atm | 6 | 140 | 46 (68) |
| 46 | 30 | 30 | 4 | air, 1 atm | 6 | 140 | 3 (7) |
| 47 | 30 | 30 | 4 | air, 10 atm | 6 | 140 | 56 (85) |
| 48 | 30 | 30 | 4 | $O_2$, 3 atm | 2 | 140 | 36 (61) |
| 49 | 30 | 30 | 4 | $O_2$, 3 atm | 4 | 140 | 51 (82) |
| 50 | 30 | 30 | 4 | $O_2$, 3 atm | 8 | 140 | 67 (94) |
| 51 | 30 | 30 | 4 | $O_2$, 3 atm | 10 | 140 | 68 (92) |
| 52 | 30 | 30 | 4 | $O_2$, 3 atm | 6 | 100 | 4 (12) |
| 53 | 30 | 30 | 4 | $O_2$, 3 atm | 6 | 120 | 51 (73) |
| 54 | 30 | 30 | 4 | $O_2$, 3 atm | 6 | 130 | 54 (83) |
| 55 | 30 | 30 | 4 | $O_2$, 3 atm | 6 | 150 | 65 (92) |

Explanations to Table 3: [a] - amount of catalyst in mol%, based on the amount of β-O-4 linkages in the lignin (see below); [b] - amount of ligand in mol%, based on the amount of β-O-4 linkages in the lignin (see below); [c] - amount of $K_2CO_3$ base in equivalents, based on the amount of β-O-4 linkages in the lignin (see below); [d] - atmosphere employed; [e] - reaction duration in hours; [f] - reaction temperature in °C; [g] - yield in % of methyl 4-(4-chlorophenoxy)-3,5-dimethoxybenzoate and in brackets the yield of all diaryl ether compounds of this reaction, in each case determined by GC and based on the amount of β-O-4 linkages in the lignin (see below).

According to the exemplary method above, several syntheses were performed using different aryl boronic acid compounds:

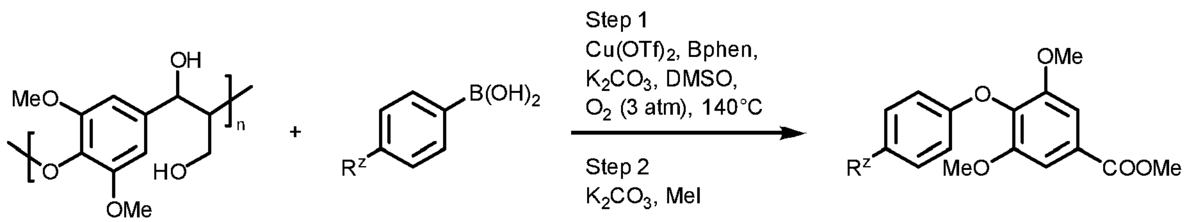


wherein the respective residues $R^z$ are listed in Table 4. The reaction conditions employed were as follows: (Step 1) Beech lignin (40 mg), boronic acid or borate ester (1.5 equiv.), $Cu(OTf)_2$ (30 mol%), Bphen (30 mol%), $K_2CO_3$ (4 equiv.), biphenyl (0.02 mmol), DMSO (2 mL), $O_2$ (3 atm), 140 °C, 6 h. (Step 2) $K_2CO_3$ (3 equiv.), MeI (10 equiv.), 25 °C, 12 h.

Table 4: Compounds synthesized with respective yields.

| Exp No. | $R^z$ [a] | Yield[b] | $^1H$ NMR[c] | $^{13}C$ NMR[d] |
|---|---|---|---|---|
| 56 | H | 66 | Fig. 11 | Fig. 12 |
| 57 | OMe | 56 | Fig. 13 | Fig. 14 |
| 58 | F | 68 | Fig. 15 | Fig. 16 |
| 59 | $C_6H_5$ | 61 | Fig. 17 | Fig. 18 |
| 60 | $CF_3$ | 71 | Fig. 19 | Fig. 20 |
| 61 | $NO_2$ | 49 | Fig. 21 | Fig. 22 |
| 62 | Br | 71 | Fig. 23 | Fig. 24 |
| 63 | $CO_2Me$ | 68 | Fig. 25 | Fig. 26 |

Explanations to Table 4: [a] - Residue $R^z$ in the synthetic scheme above; [b] - Yield of the major diaryl ether product as

depicted in the synthetic scheme, determined by GC (see below); [c] - Figure containing $^{1}H$ NMR spectrum of the major diaryl ether product (see synthetic scheme above) in $CDCl_3$; [d] - Figure containing $^{13}C$ NMR spectrum of the major diaryl ether product (see synthetic scheme above) in $CDCl_3$.

Analytical Methods

Qualitative and quantitative analysis of the crude products was performed by gas chromatography (GC) (Agilent 7890B, equipped with mass detector (Agilent 7000C) equipped with a hydrogen flame-ionization detector (FID) and HP-5 nonpolar column. Helium was used as the carrier gas. The GC yield was determined based on internal standard curves (biphenyl) and areas of integrated peak area.

$^{1}H$ and $^{13}C$ NMR spectra were recorded on a Bruker Avance III HD 400 instrument equipped with a 5 mm BBFO probe. DMSO-$d_6$ or $CDCl_3$ were used as solvent. Short-range $^{13}C$-$^{1}H$ correlation NMR spectra (HSQC) were recorded on a 500 MHz spectrometer (Bruker AVANCENEO-500). HSQC spectra of the original reaction mixture were recorded in DMSO-$d_6$, used as the reaction solvent. The reaction mixture was dried with 4A molecular sieve and centrifuged to remove inorganic salts.

Quantitative methods

Determination of the concentration of β-O-4 linkages was conducted using HSQC NMR spectroscopy with trioxane as an internal standard.

Briefly, beech lignin was extracted with dioxane from beech wood sawdust, leaving carbohydrates as solid residues (see also Lancefield, C. S.; Ojo, O. S.; Tran, F.; Westwood, N. J., Isolation of Functionalized Phenolic Monomers through Selective Oxidation and CO Bond Cleavage of the β-O-4 Linkages in Lignin. Angew. Chem. Int. Ed. 2015, 54, 258-262.). In the lignin polymer, the aromatic rings are connected by serval types of alkyl side chain. The different connections of the aromatic rings can be identified by short-range $^{13}C$-$^{1}H$ correlation (HSQC) NMR spectroscopy. For example, the beech lignin structure comprises syringyl and guaiacyl aromatic rings, together with their main alkyl side chains, β-O-4 (A) and β-β (B) linkages, which are clearly identified by HSQC NMR spectroscopy.

Determination of the concentration of β-O-4 linkages by quantitative $^{13}C$ NMR spectroscopy would be hindered by overlapping signals from other alkyl linkages including the β-β and α-O-4 linkages.[2-3] Thus, HSQC NMR spectroscopy was employed to determine the concentration of the β-O-4 linkages using trioxane as an internal standard (chemical shift of trioxane: $\delta_H/\underline{\delta_C}$: 5.1/93.4 ppm). Here, the integrals of HSQC NMR signals of different functional groups are linearly related to their amounts (Sette M., Lange H., Crestini C., QUANTITATIVE HSQC ANALYSES OF LIGNIN: A PRACTICAL COMPARISON. Comput. Struct. Biotechnol. J. 2013, 6, e201303016; Heikkinen, S.; Toikka, M. M.; Karhunen, P. T.; Kilpeläinen, I. A., Quantitative 2D HSQC (Q-HSQC) via Suppression of J-Dependence of Polarization Transfer in NMR Spectroscopy: Application to Wood Lignin. J. Am. Chem. Soc. 2003, 125, 4362-4367).

Using the model compound 1-(3,4-dimethoxyphenyl)-2-(2-methoxyphenoxy)propane-1,3-diol having the structure (i)

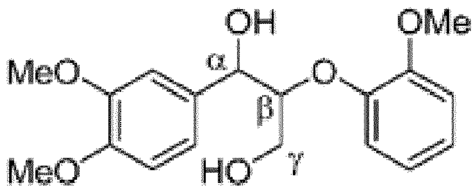

(i),

the molar correlation coefficient between the amount of β-O-4 linkages and the integral of the peak in the HSQC NMR spectrum was determined. In the structure (i) above, the α-, β-, and γ-positions are labelled. Sections of the HSQC NMR spectra of the model compound 1-(3,4-dimethoxyphenyl)-2-(2-methoxyphenoxy)propane-1,3-diol together with trioxane at different known concentrations and molar ratios are shown in Figures 1a to 1c.

For the determination of the molar correlation factor $f_{\beta\text{-O-4}}$ for the amount of β-O-4 linkages, the integral of the peak $A_\alpha$ at $\delta_H/\underline{\delta_C}$: 4.9/72.3 ppm (the integral being referred to as $S_{\beta\text{-O-4}}$) and the integral of the trioxane peak at $\delta_H/\underline{\delta_C}$: 5.1/93.4 ppm (the integral being referred to as $S_{trioxane}$) in the HSQC NMR spectra in Figures 1a to 1c were used. In Figure 1a, 19.2 mg 1-(3,4-dimethoxyphenyl)-2-(2-methoxyphenoxy)propane-1,3-diol and 5.1 mg trioxane in 0.5 mL DMSO-$d_6$ were used. In Figure 1b, 10.9 mg 1-(3,4-dimethoxyphenyl)-2-(2-methoxyphenoxy)propane-1,3-diol and 5.0 mg trioxane in 0.5 mL DMSO-$d_6$ were used. In Figure 1c, 7.4 mg 1-(3,4-dimethoxyphenyl)-2-(2-methoxyphenoxy)propane-1,3-diol and 6.9 mg trioxane in 0.5 mL DMSO-$d_6$ were used.

In the three HSQC NMR spectra of Figure 1a to 1c, the integrals $S_{\beta\text{-O-4}}$ and Strioxane were determined, with the value of $S_{trioxane}$ being adjusted to 100. For each of the HSQC NMR spectra in Figure 1a to 1c, the ratio ($6*S_{\beta\text{-O-4}}/S_{trioxane}$) was calculated and plotted against the ratio Mi/Mt, wherein Mi is the molar amount of 1-(3,4-dimethoxyphenyl)-2-(2-methoxyphenoxy)propane-1,3-diol and Mt is the molar amount of trioxane employed in the solutions for the respective HSQC NMR spectrum. The factor 6 in the ratio of the integrals is due to the different amounts of protons giving rise to the peaks for $A_\alpha$ and trioxane. Linear regression analysis of the resulting points in the graph and additionally the origin (0;0) of the graph was performed to reveal a straight line through these points and through the origin of the graph. This is also further described in Sette M., Lange H., Crestini C., QUANTITATIVE HSQC ANALYSES OF LIGNIN: A PRACTICAL COMPARISON. Comput. Struct. Biotechnol. J. 2013, 6, e201303016 and in Heikkinen, S.; Toikka, M. M.; Karhunen, P. T.; Kilpeläinen, I. A., Quantitative 2D HSQC (Q-HSQC) via Suppression of J-Dependence of Polarization Transfer in NMR Spectroscopy: Application to Wood Lignin.J.Am.Chem.Soc. 2003, 125, 4362-4367. The slope factor of this straight line amounted to 2.9613 and was used as the molar correlation factor $f_{\beta\text{-O-}4}$.

With the molar correlation factor from the model compound as determined above, the molar amount of β-O-4 linkages per gram of extracted beech lignin (see above) was determined as follows. 54.6 mg of the extracted beech lignin and 4.5 mg of trioxane were dissolved in 0.5 mL DMSO-$d_6$, and an HSQC NMR spectrum was recorded. A section of the HSQC NMR spectrum of extracted beech lignin is shown in Figure 2 along with the structure of the repeat units found in beech lignin and the corresponding peak assignment in the HSQC NMR spectrum. The peak of the β-O-4 linkages in the HSQC NMR spectrum is denoted $A_\alpha$ in Figure 2, as above. In the HSQC NMR spectrumbereich liegen, the integral $S_{\beta\text{-O-4}}$ of the peak $A_\alpha$ and the integral Strioxane of the trioxane peak were determined, with the value of $S_{trioxane}$ being adjusted to 100, resulting in a value of 13.2 for the integral $S_{\beta\text{-O-4}}$. The molar amount of β-O-4 linkages per gram in the extracted beech lignin was then calculated as follows:

The molar amount of trioxane ($n_{trioxane}$) was calculated by dividing the mass of trioxane ($m_{trioxane}$) by the molecular weight of trioxane (Mw(trioxane)):

$$n_{trioxane} = \frac{m_{trioxane}}{M_w(trioxane)} = \frac{4.5\ \mathrm{mg}}{90.1} = 0.05\ \mathrm{mmol}$$

The molar amount of β-O-4 linkages ($M'_{\beta\text{-O-4}}$) in the analyzed beech lignin sample (54.6 mg) was calculated using the integrals $S_{\beta\text{-O-4}}$ and Strioxane (see above, also for the factor 6) as well as the molar correlation factor $f_{\beta\text{-O-4}}$ and the molar amount of trioxane as:

$$M'_{\beta\text{-}O\text{-}4} = \frac{6 * S_{\beta\text{-}O\text{-}4}}{S_{trioxane} * f_{\beta\text{-}O\text{-}4}} \times n_{\mathrm{trioxane}} = 0.0134\ \mathrm{mmol}/54.6\ \mathrm{mg}$$

Then, the molar amount of β-O-4 linkages ($M_{\beta\text{-O-4}}$) in beech lignin per gram was calculated using the mass of the lignin sample (54.6 mg) as:

$$M_{\beta\text{-}O\text{-}4} = M'_{\beta\text{-}O\text{-}4} \times \frac{1000}{m_{lignin}} = 0.245\ \mathrm{mmol/g}$$

According to this method, the amounts of other alkyl side linkages in lignin and the amounts of β-O-4 linkages in different kinds of lignin sources may also be quantified.

The yield (%) of ether products is based on the amount of the β-O-4 linkages in the lignin as follows:

$$Yield_{ether} = \frac{n_{ether}}{m_{lignin} \times M_{\beta\text{-}O\text{-}4}} \times 100\%$$

$n_{ether}$ was determined by GC based on internal standard curves and areas of integrated peak area.

$n_{ether}$: mol of ether products

## Claims

**1.** Diaryl ether compound having the structure (I)

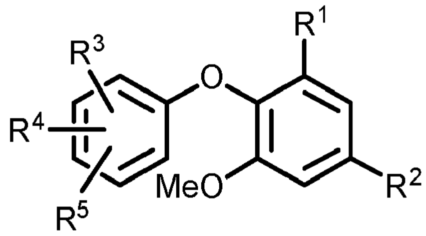

(I),

wherein

$R^1$ is H or -OMe,
$R^2$ is -CHO, $-CO_2H$, $C_1$-$C_4$-alkoxycarbonyl, or $C_3$-$C_6$-cycloalkoxycarbonyl,
$R^3$, $R^4$, and $R^5$ are independently of each other H, halogen, halogenated $C_1$-$C_4$-alkyl, $C_5$-$C_{10}$-aryl, in particular phenyl, nitro, $C_1$-$C_4$-alkoxy, $C_1$-$C_4$-alkoxycarbonyl,
or $C_3$-$C_6$-cycloalkoxycarbonyl.

**2.** Diaryl ether compound according to claim 1, wherein $R^2$ is -CHO, $-CO_2H$, or $-CO_2Me$.

**3.** Diaryl ether compound according to any one of claims 1 or 2, wherein $R^3$, $R^4$, and $R^5$ are independently of each other H, F, Cl, Br, perfluorinated $C_1$-$C_4$-alkyl, preferably $-CF_3$, $-NO_2$, -OMe, or $-CO_2Me$.

**4.** Diaryl ether compound according to any one of claims 1 to 3, wherein $R^3$ is as defined in any one of claims 1 to 3 and $R^4$ and $R^5$ are H.

**5.** Diaryl ether compound according to any one of claims 1 to 4, wherein $R^3$ is in para position.

**6.** Method for the manufacture of a diaryl ether compound according to any one of claims 1 to 5, wherein lignin is reacted with an aryl boronic compound having the structure (II)

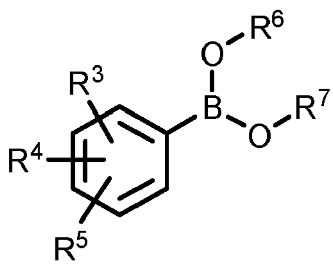

(II),

wherein

$R^3$, $R^4$, and $R^5$ are as defined in any one of claims 1 to 5,
$R^6$ and $R^7$ are independently of each other H, $C_1$-$C_4$-alkyl, or $R^6$ and $R^7$ together with the oxygen atoms and the boron atom form a 5- or 6-membered ring optionally substituted with1 to 4 $C_1$-$C_3$-alkyl substituents.

**7.** Method according to claim 6, wherein the lignin is reacted with the aryl boronic compound having the structure (II)

in the presence of a transition metal salt, preferably a copper salt, a palladium salt, or a ruthenium salt, more preferably a copper salt, even more preferred copper chloride, copper nitrate, or copper triflate, most preferred copper triflate.

**8.** Method according to any one of claims 6 or 7, wherein the lignin is reacted with the aryl boronic compound having the structure (II) in the presence of a nitrogen-containing aromatic ligand, preferably a nitrogen-containing aromatic ligand selected from the group consisting of

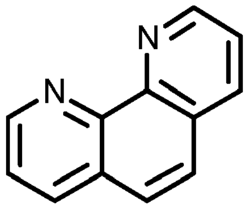
(L-I),

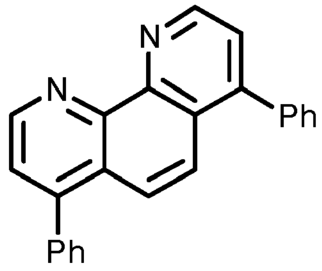
(L-II), and

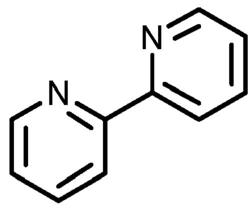
(L-III),

more preferably in the presence of

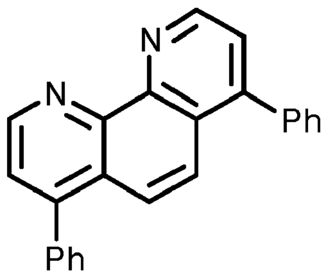
(L-II).

**9.** Method according to any one of claims 6 to 8, wherein the lignin is reacted with the aryl boronic compound having the structure (II) under oxidizing conditions, preferably in the presence of oxygen.

**10.** Method according to any one of claims 6 to 9, wherein the lignin is reacted with the aryl boronic compound having the structure (II) in the presence of a base, preferably an inorganic base, more preferably, $Na_2CO_3$, $K_2CO_3$, and/or $Cs_2CO_3$, and/or in the presence of a solvent, preferably selected from the group consisting of dimethylformamide, dimethylacetamide, N-methylpyrrolidone, dimethylsulfoxide, acetonitrile, water, and mixtures thereof, more preferably dimethylsulfoxide.

**11.** Method according to any one of claims 6 to 10, wherein the lignin is reacted with the aryl boronic compound having the structure (II) at a temperature from 80 to 200°C, preferably from 100 to 180°C, more preferably from 120 to 160°C, most preferably from 130 to 150°C.

**12.** Method according to any one of claims 6 to 11, wherein the aryl boronic compound having the structure (II) is employed in an amount of 1.0 to 3.0 equivalents, preferably 1.0 to 2.5 equivalents, more preferably 1.0 to 2.0 equivalents, based on the total amount of β-O-4 linkages in the lignin.

**13.** Method according to any one of claims 6 to 12, wherein $R^6$ and $R^7$ are H or $R^6$ and $R^7$ form together with the oxygen atoms and the boron atom a pinakolboryl residue or a neopentylglycolboryl residue.

**14.** Use of an aryl boronic compound having the structure (II)

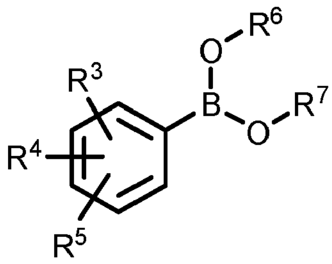

(II),

in the conversion of lignin to a diaryl ether compound,
wherein $R^3$, $R^4$, and $R^5$ are as defined in any one of claims 1 to 5 and $R^6$ and $R^7$ are as defined in claims 6 or 13.

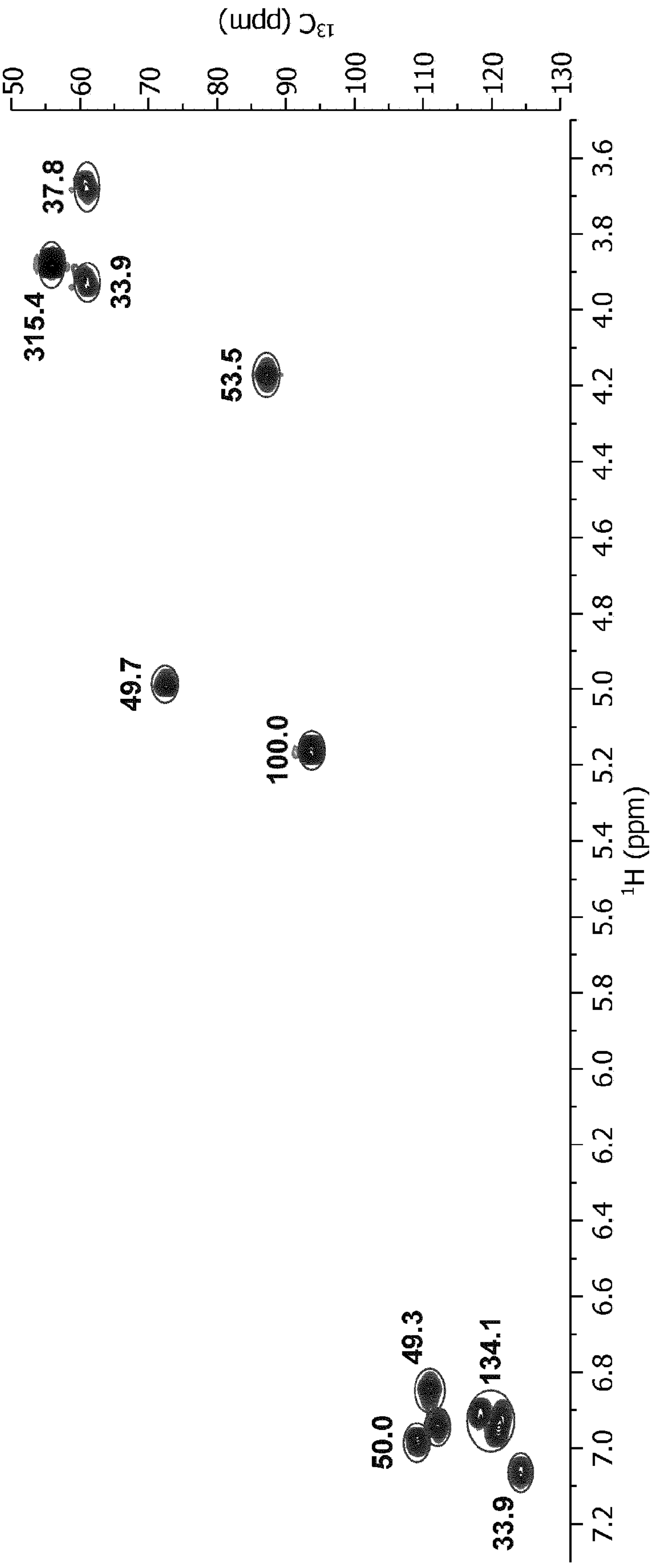


Fig.1a

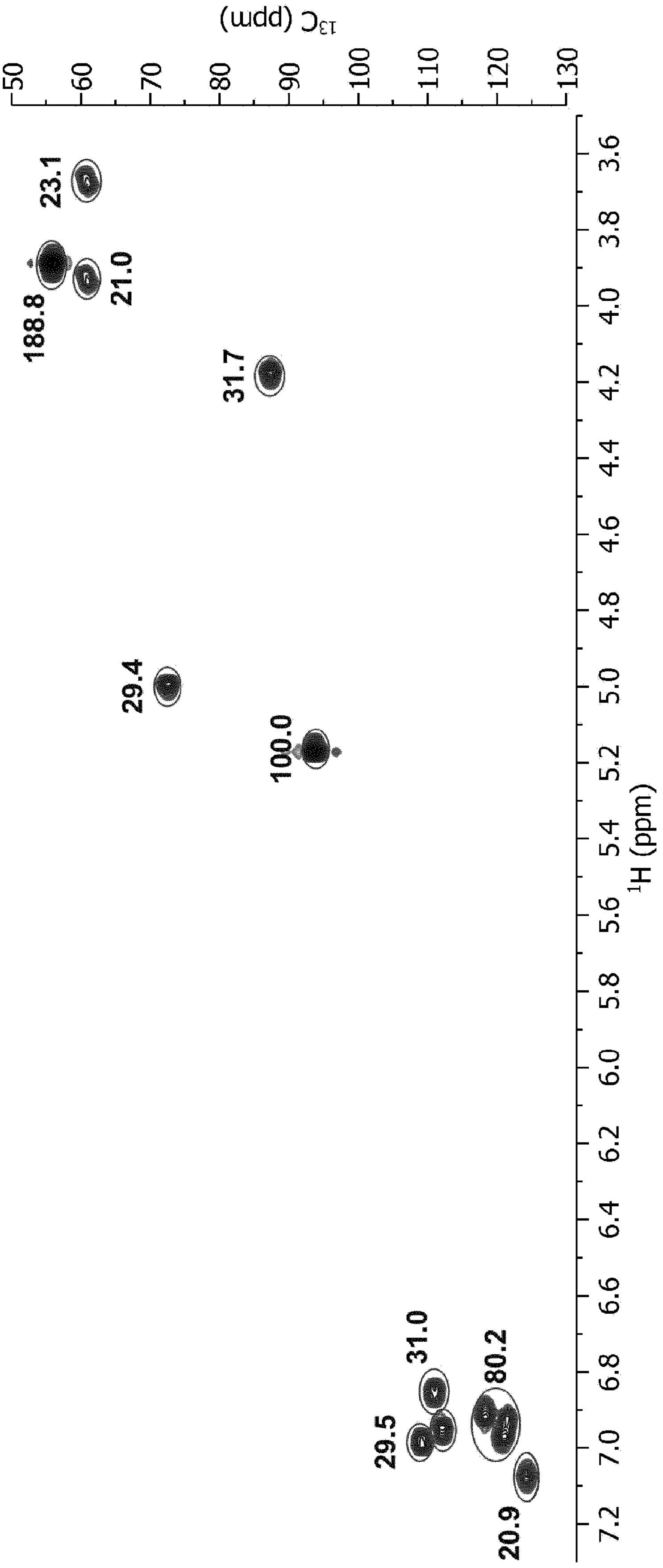


Fig.1b

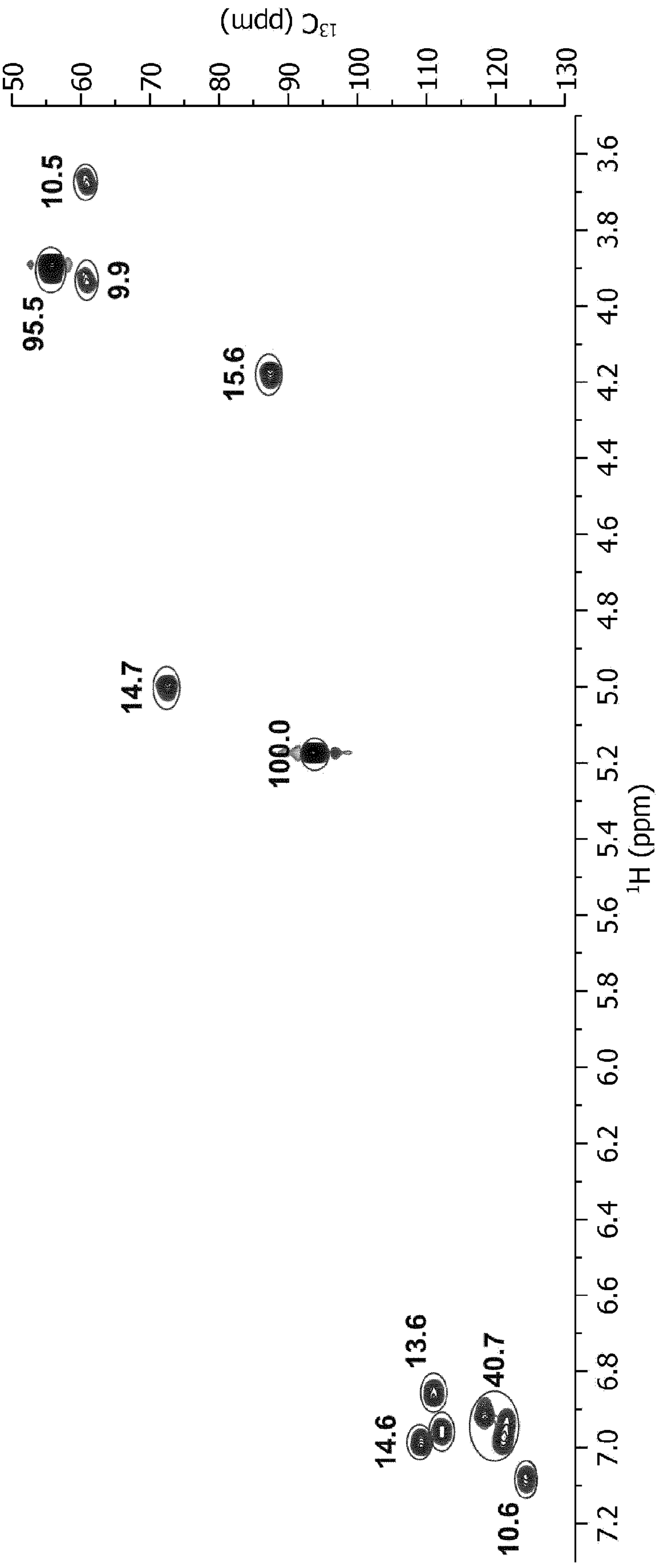
13C (ppm)
50
60
70
80
90
100
110
120
130
1H (ppm)
3.6
3.8
4.0
4.2
4.4
4.6
4.8
5.0
5.2
5.4
5.6
5.8
6.0
6.2
6.4
6.6
6.8
7.0
7.2
10.5
95.5
9.9
15.6
14.7
100.0
13.6
14.6
40.7
10.6

Fig.1c

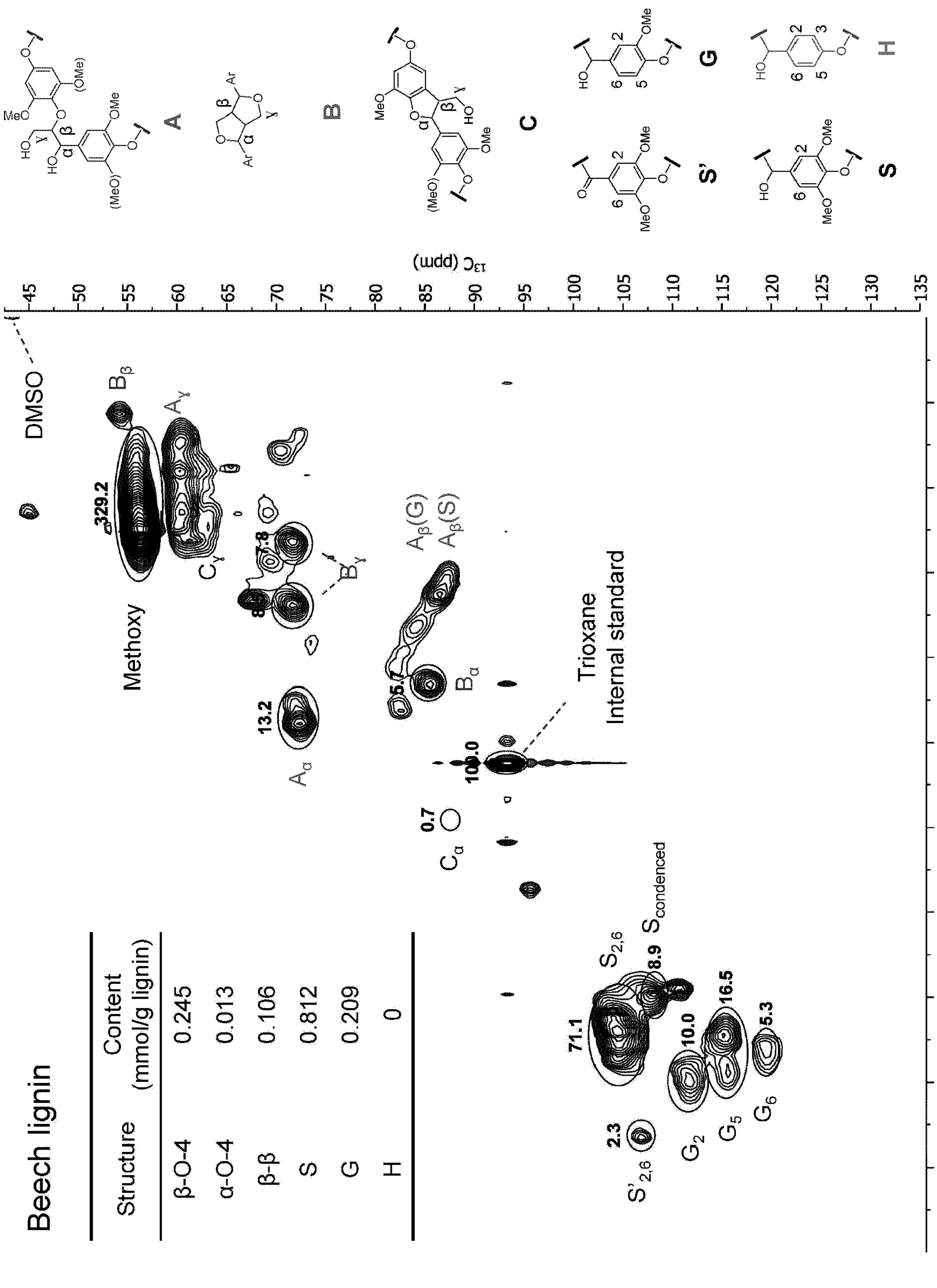


| Structure | Content (mmol/g lignin) |
|---|---|
| β-O-4 | 0.245 |
| α-O-4 | 0.013 |
| β-β | 0.106 |
| S | 0.812 |
| G | 0.209 |
| H | 0 |

Fig.2

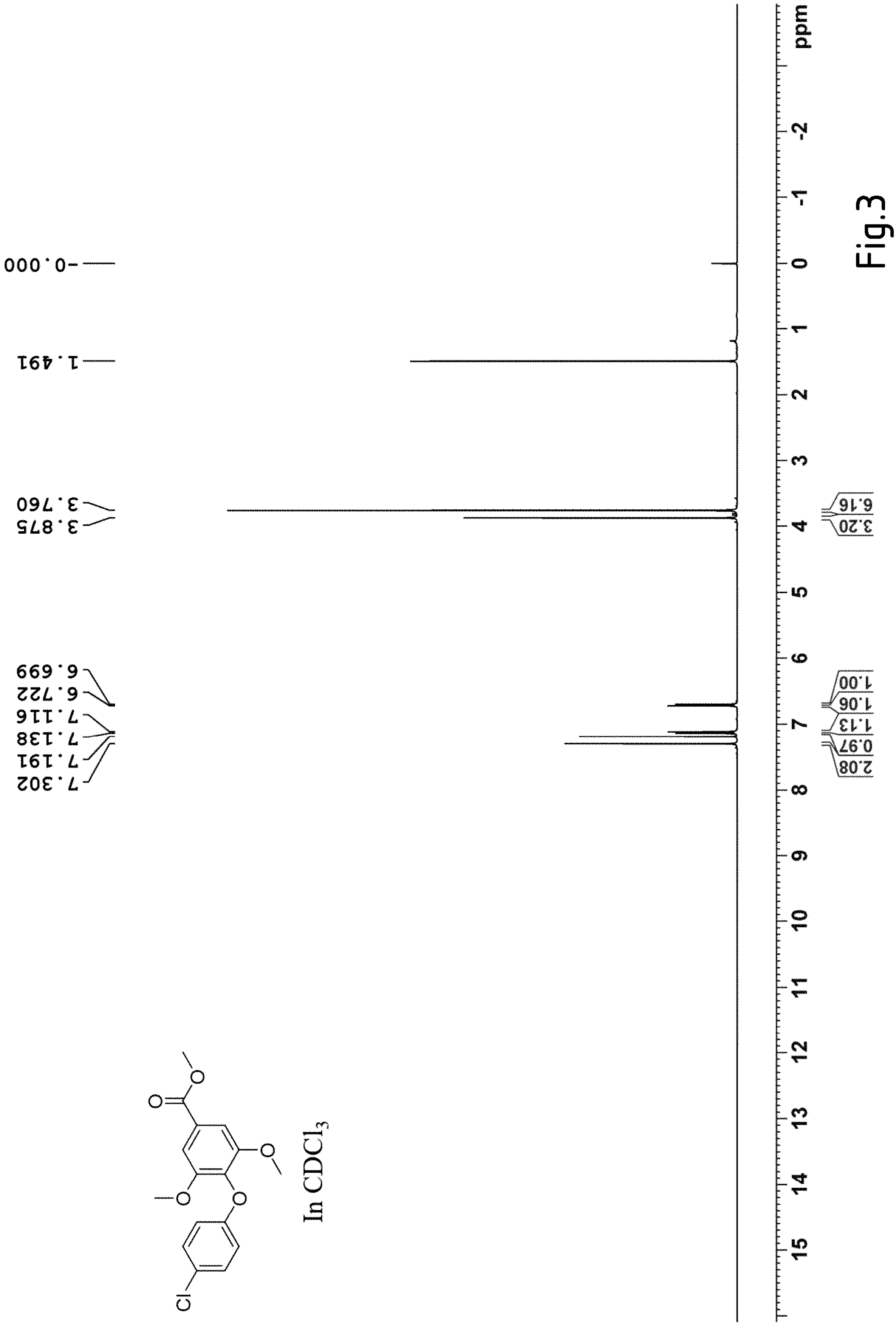
-0.000
1.491
3.760
3.875
6.699
6.722
7.116
7.138
7.191
7.302
6.16
3.20
1.00
1.06
1.13
0.97
2.08
ppm
In $CDCl_3$

Fig.3

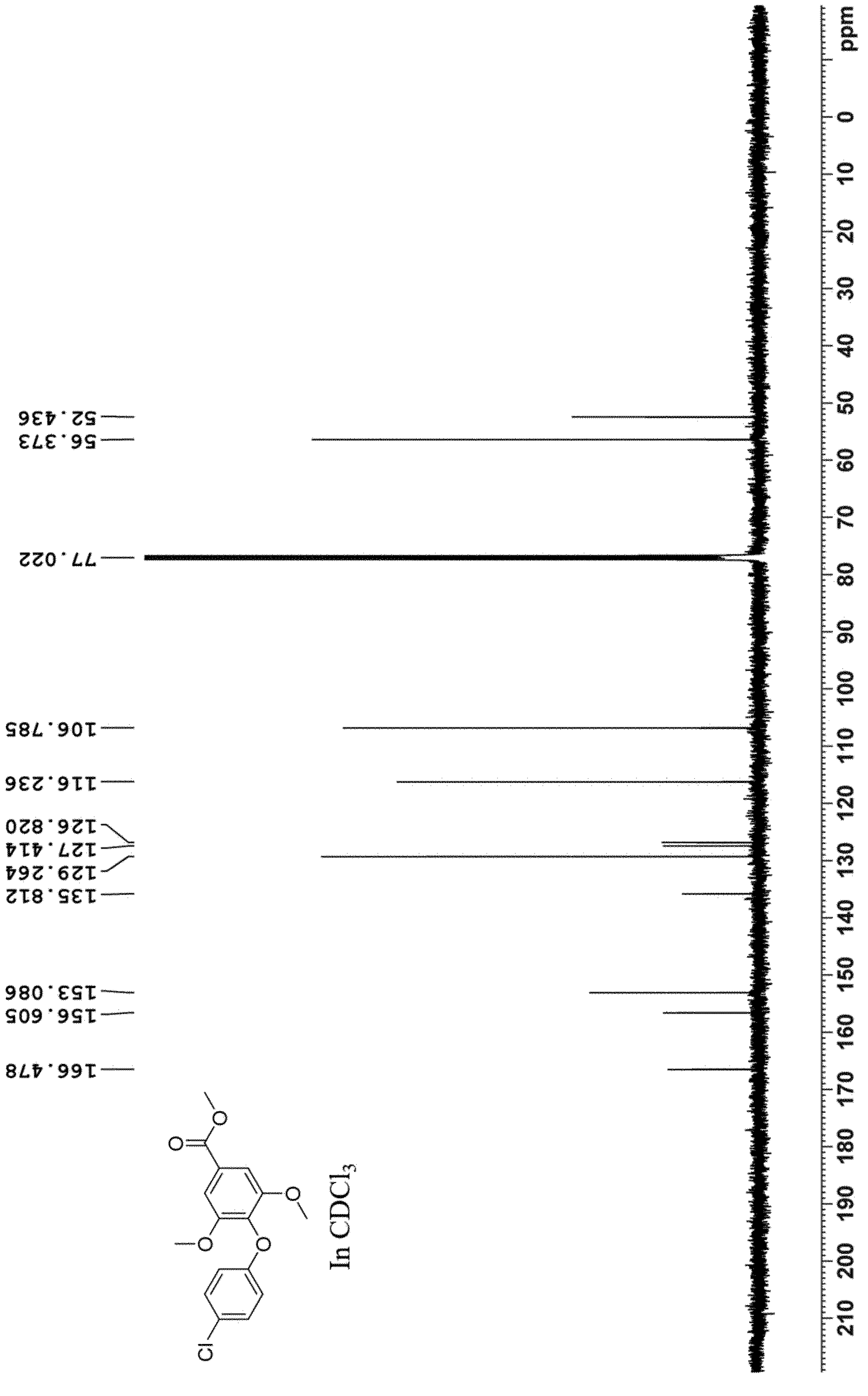
52.436
56.373
77.022
106.785
116.236
126.820
127.414
129.264
135.812
153.086
156.605
166.478
In $CDCl_3$
ppm
0
10
20
30
40
50
60
70
80
90
100
110
120
130
140
150
160
170
180
190
200
210

Fig.4

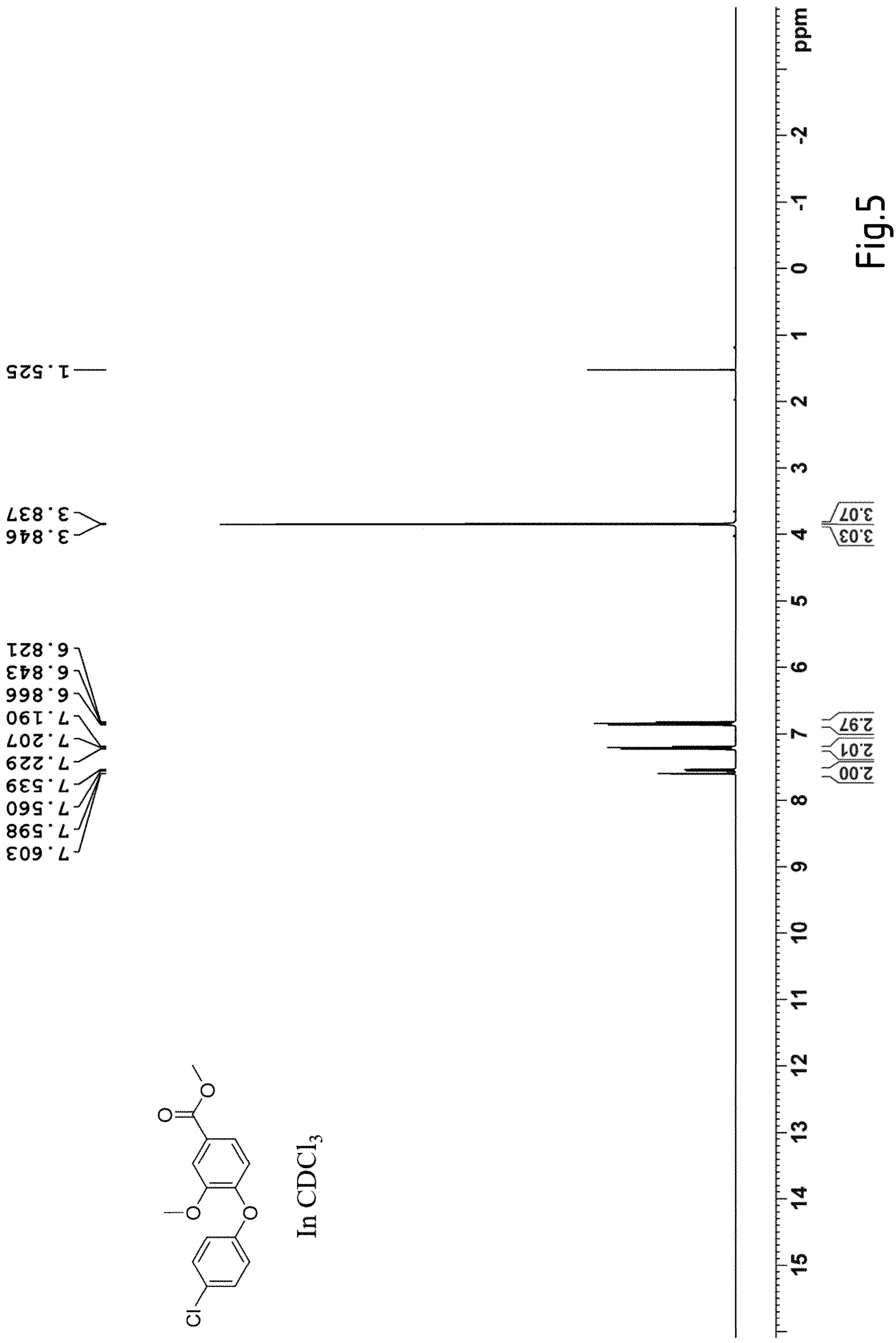
1.525
3.837
3.846
6.821
6.843
6.866
7.190
7.207
7.229
7.539
7.560
7.598
7.603
3.07
3.03
2.97
2.01
2.00
ppm
-2
-1
0
1
2
3
4
5
6
7
8
9
10
11
12
13
14
15
In $CDCl_3$

Fig.5

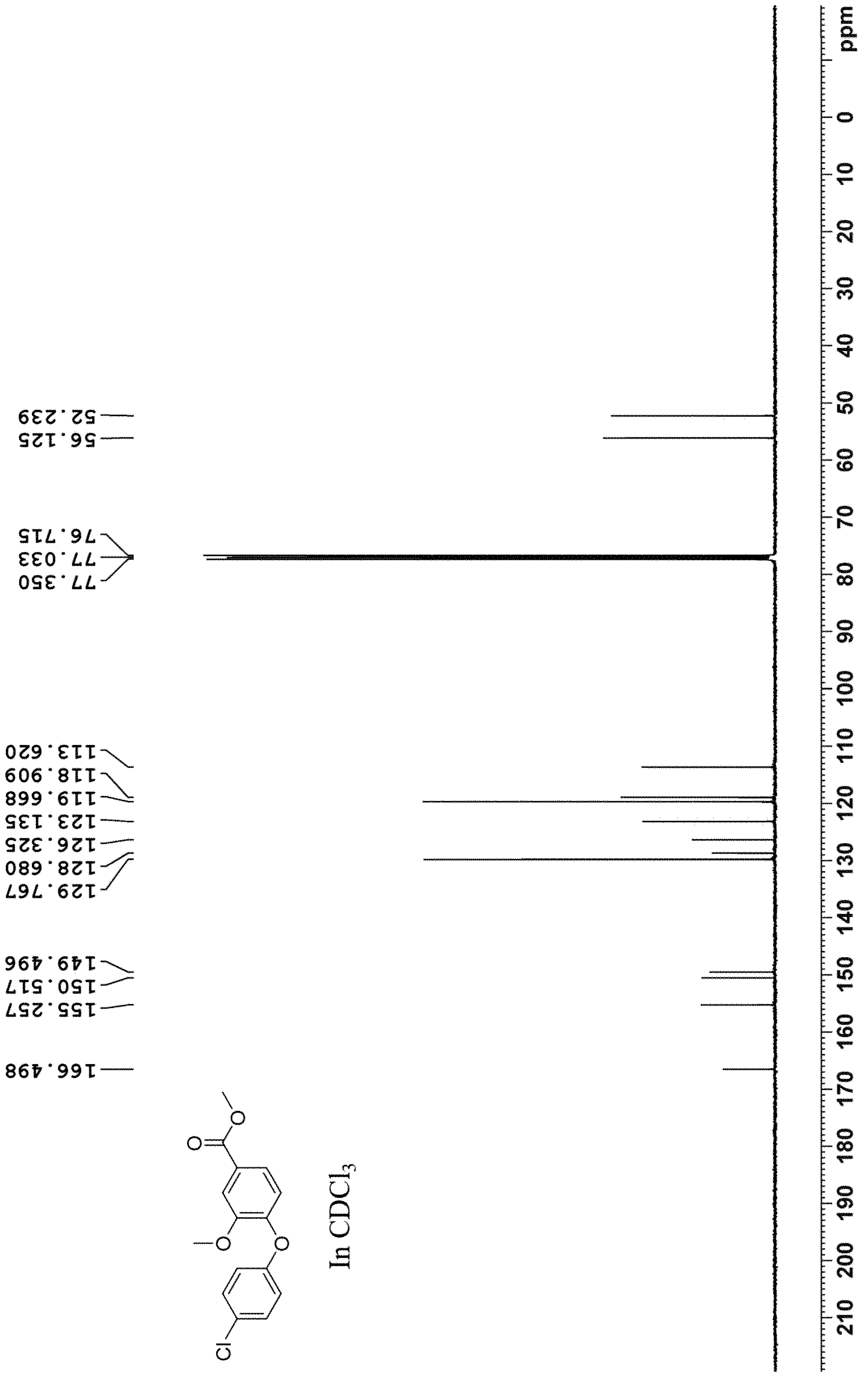
52.239
56.125
76.715
77.033
77.350
113.620
118.909
119.668
123.135
126.325
128.680
129.767
149.496
150.517
155.257
166.498
In $CDCl_3$
ppm
0
10
20
30
40
50
60
70
80
90
100
110
120
130
140
150
160
170
180
190
200
210

Fig.6

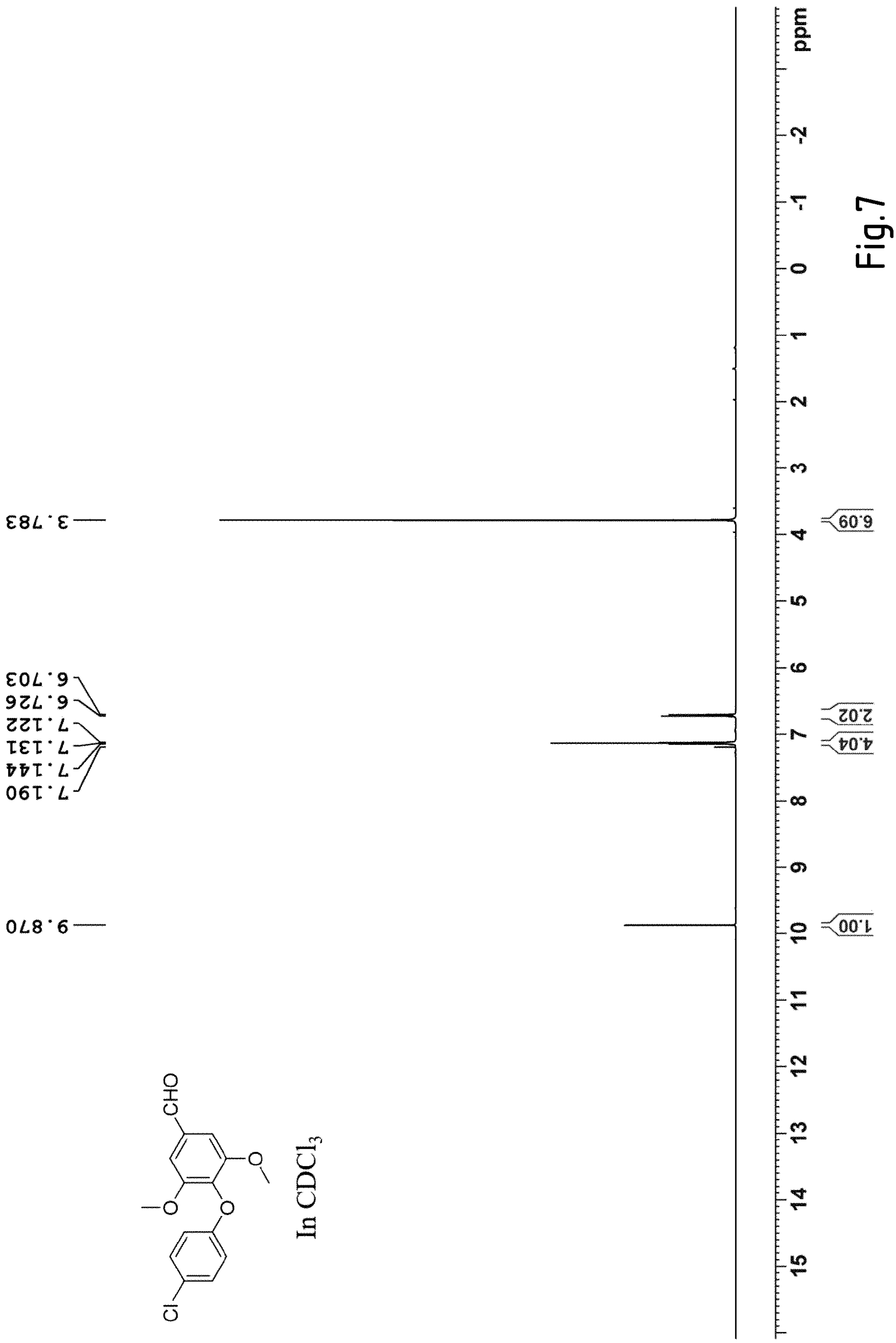
3.783
6.703
6.726
7.122
7.131
7.144
7.190
9.870
6.09
2.02
4.04
1.00
ppm
In $CDCl_3$

Fig.7

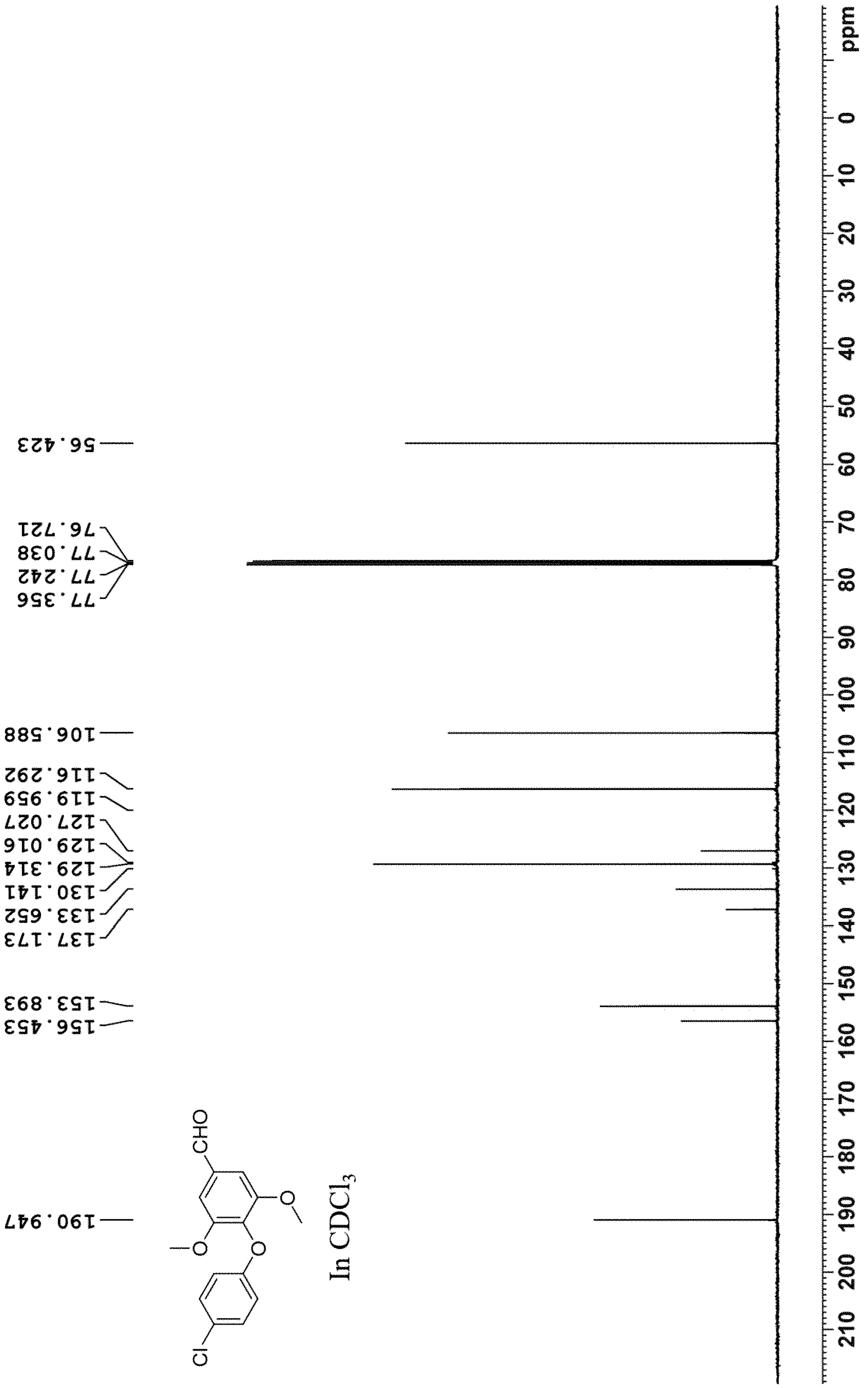
56.423
76.721
77.038
77.242
77.356
106.588
116.292
119.959
127.027
129.016
129.314
130.141
133.652
137.173
153.893
156.453
190.947
In $CDCl_3$
ppm
0
10
20
30
40
50
60
70
80
90
100
110
120
130
140
150
160
170
180
190
200
210

Fig.8

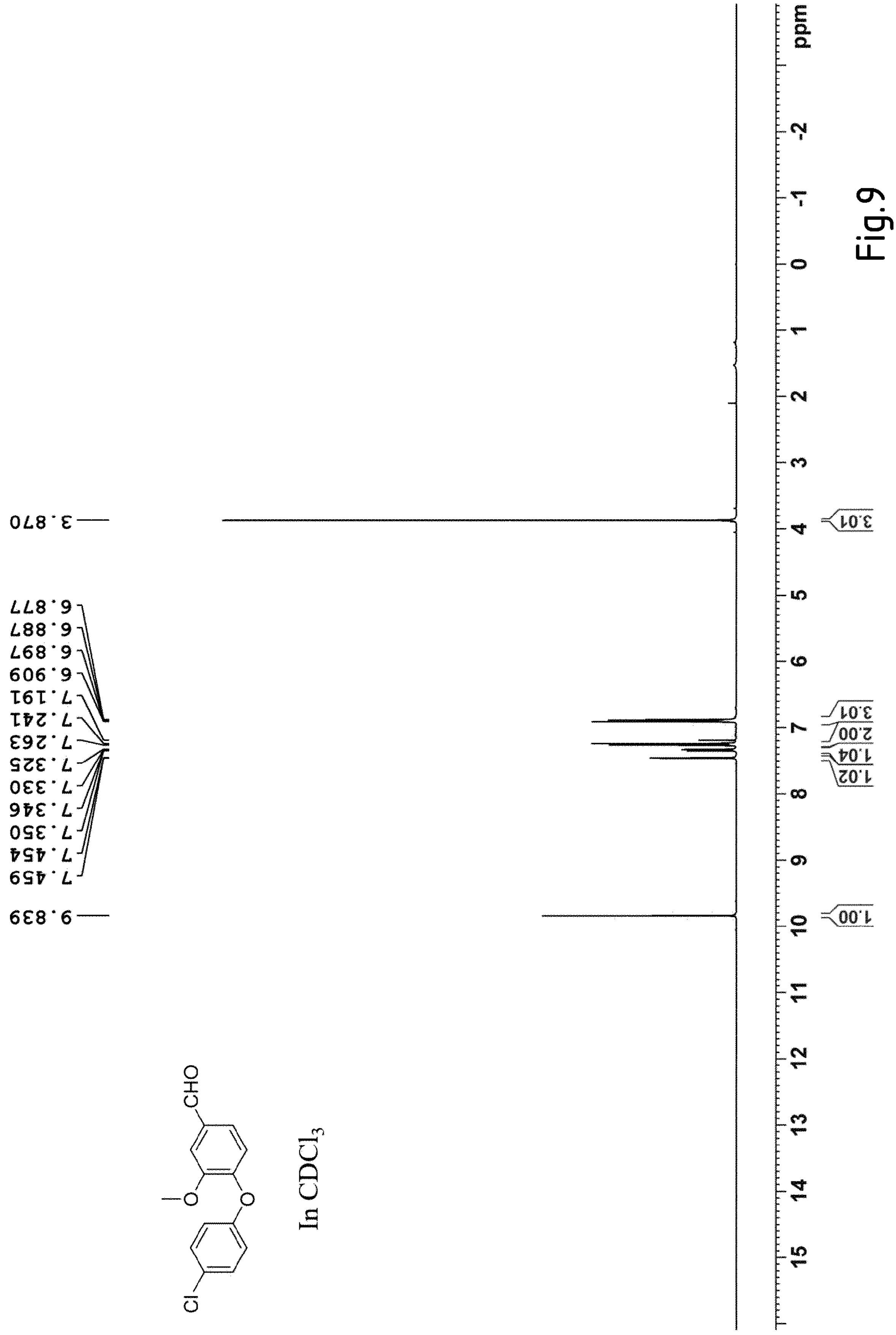
3.870
6.877
6.887
6.897
6.909
7.191
7.241
7.263
7.325
7.330
7.346
7.350
7.454
7.459
9.839
In CDCl3
3.01
3.01
2.00
1.04
1.02
1.00
ppm

Fig.9

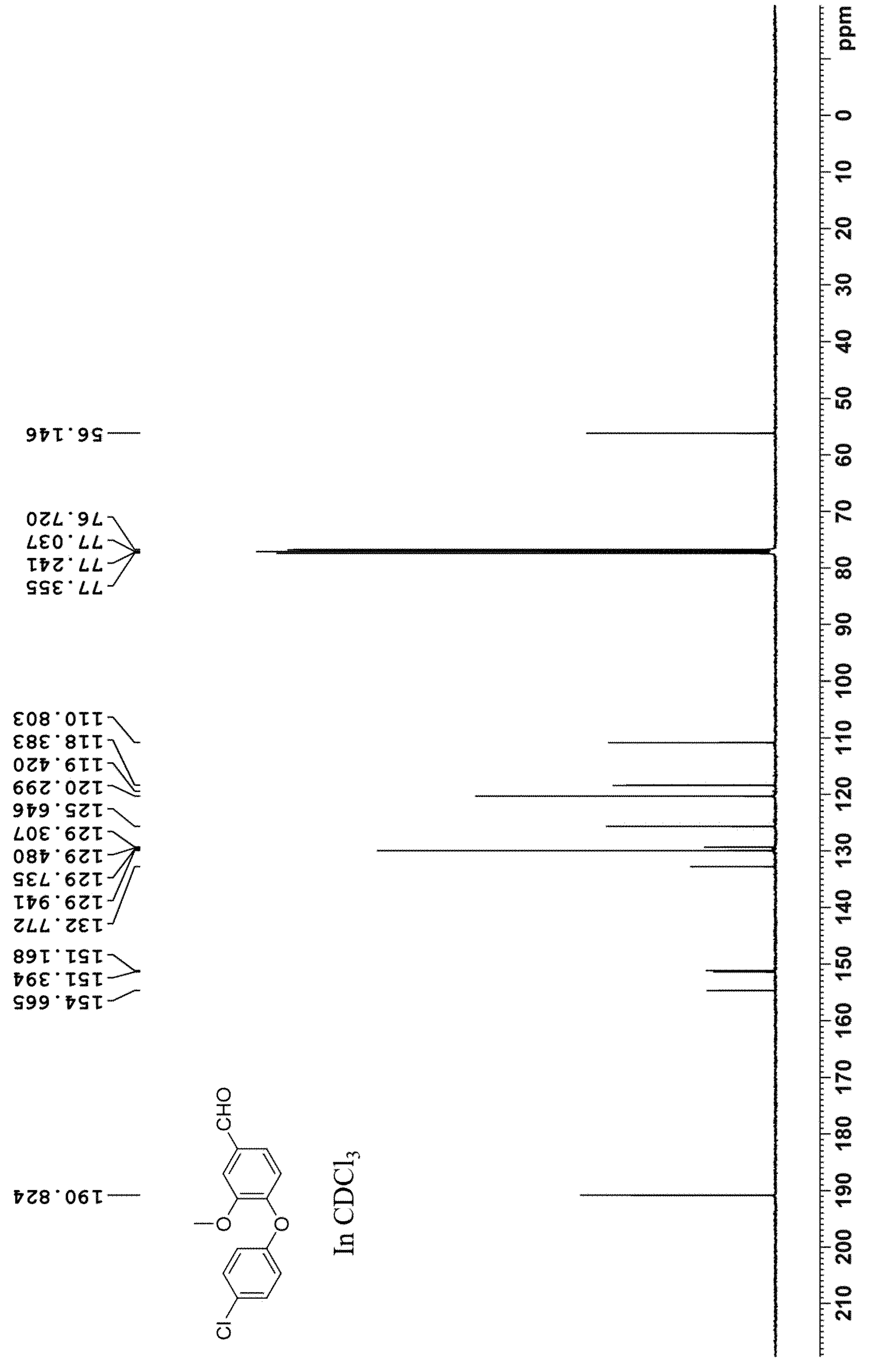
56.146
76.720
77.037
77.241
77.355
110.803
118.383
119.420
120.299
125.646
129.307
129.480
129.735
129.941
132.772
151.168
151.394
154.665
190.824
In CDCl3
ppm
0
10
20
30
40
50
60
70
80
90
100
110
120
130
140
150
160
170
180
190
200
210

Fig.10

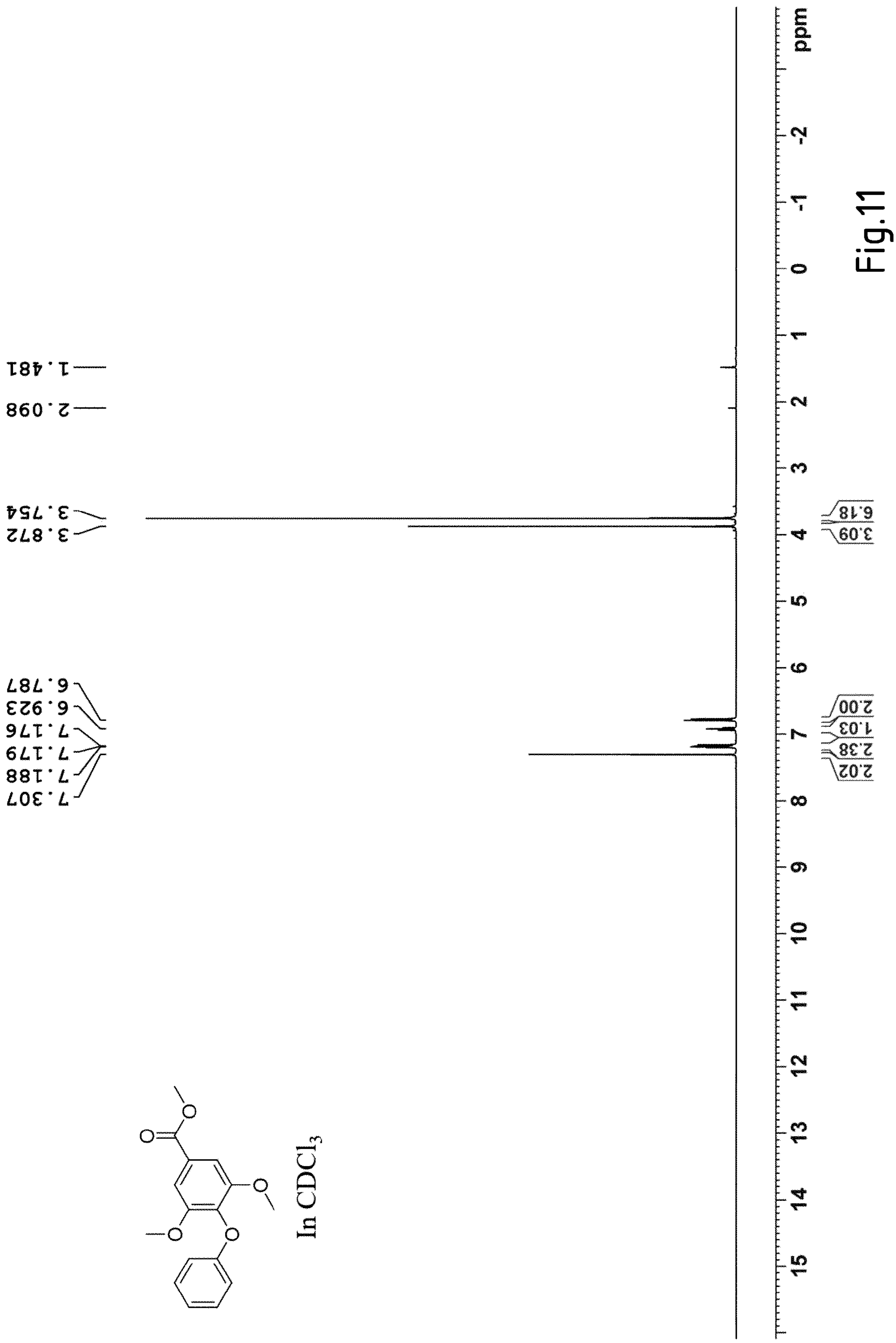

1.481
2.098
3.754
3.872
6.787
6.923
7.176
7.179
7.188
7.307
6.18
3.09
2.00
1.03
2.38
2.02
ppm
-2
-1
0
1
2
3
4
5
6
7
8
9
10
11
12
13
14
15
In $CDCl_3$


Fig.11

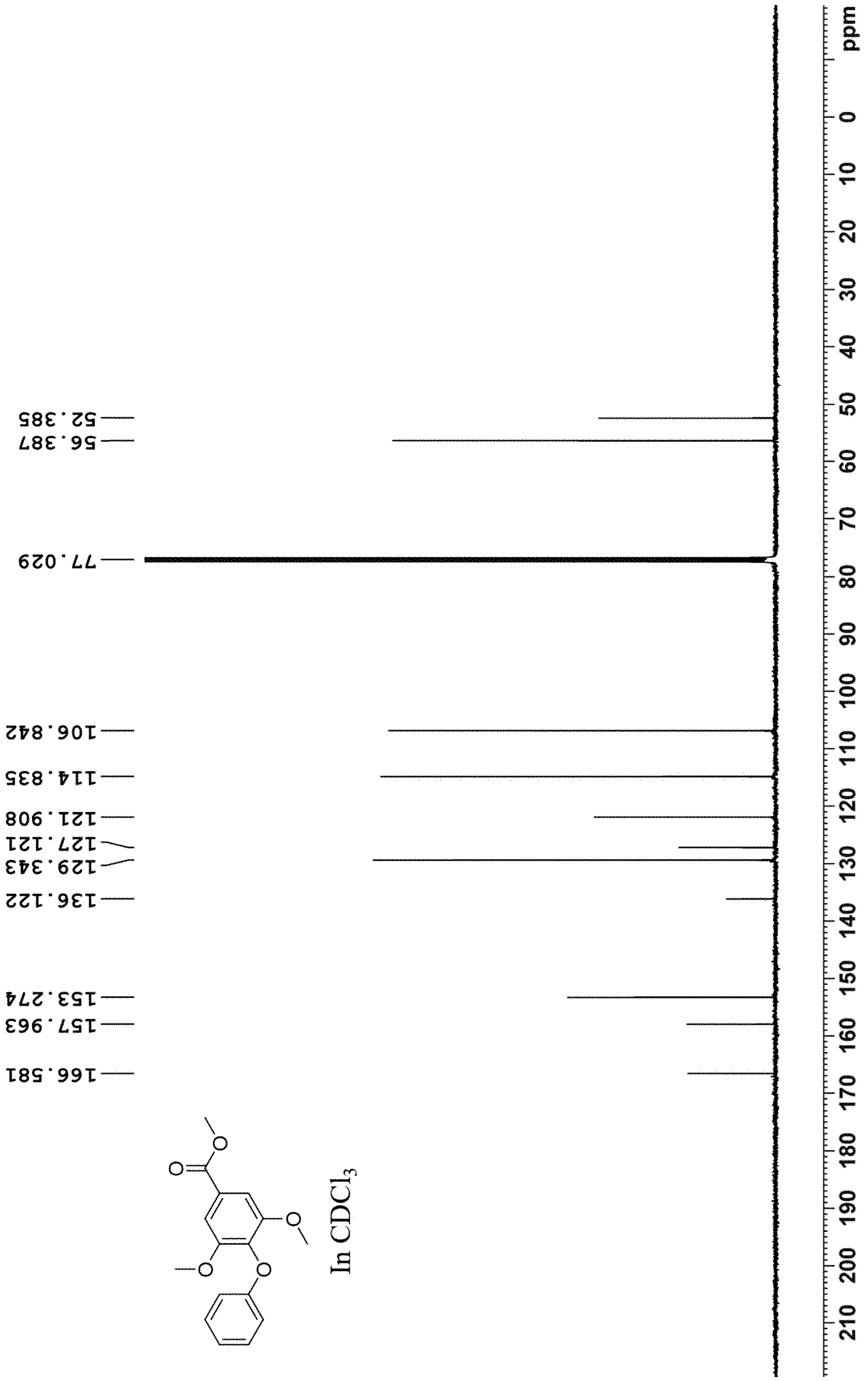
52.385
56.387
77.029
106.842
114.835
121.908
127.121
129.343
136.122
153.274
157.963
166.581
In $CDCl_3$
ppm
0
10
20
30
40
50
60
70
80
90
100
110
120
130
140
150
160
170
180
190
200
210

Fig.12

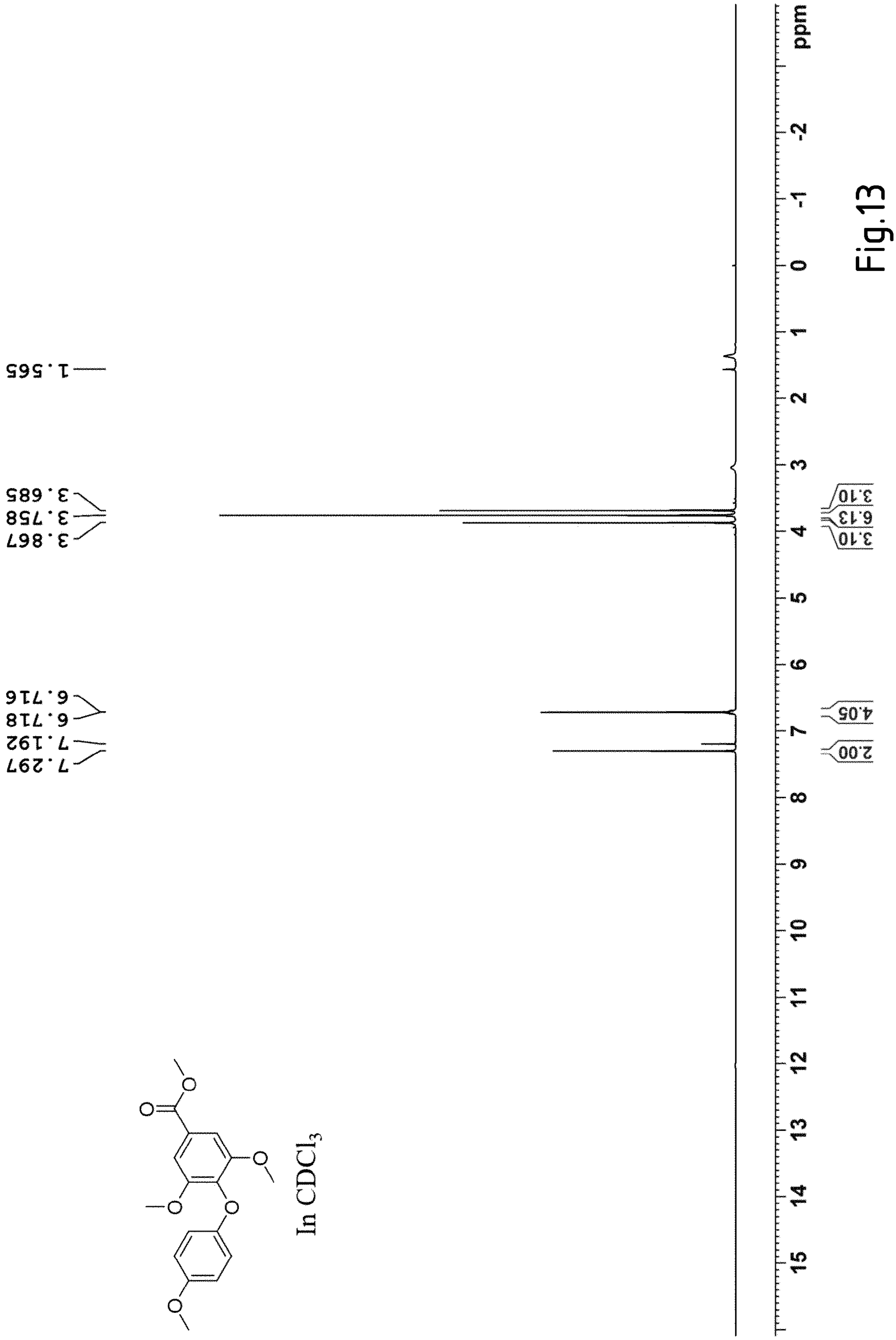
1.565
3.685
3.758
3.867
6.716
6.718
7.192
7.297
3.10
6.13
3.10
4.05
2.00
In $CDCl_3$
ppm
-2
-1
0
1
2
3
4
5
6
7
8
9
10
11
12
13
14
15

Fig.13

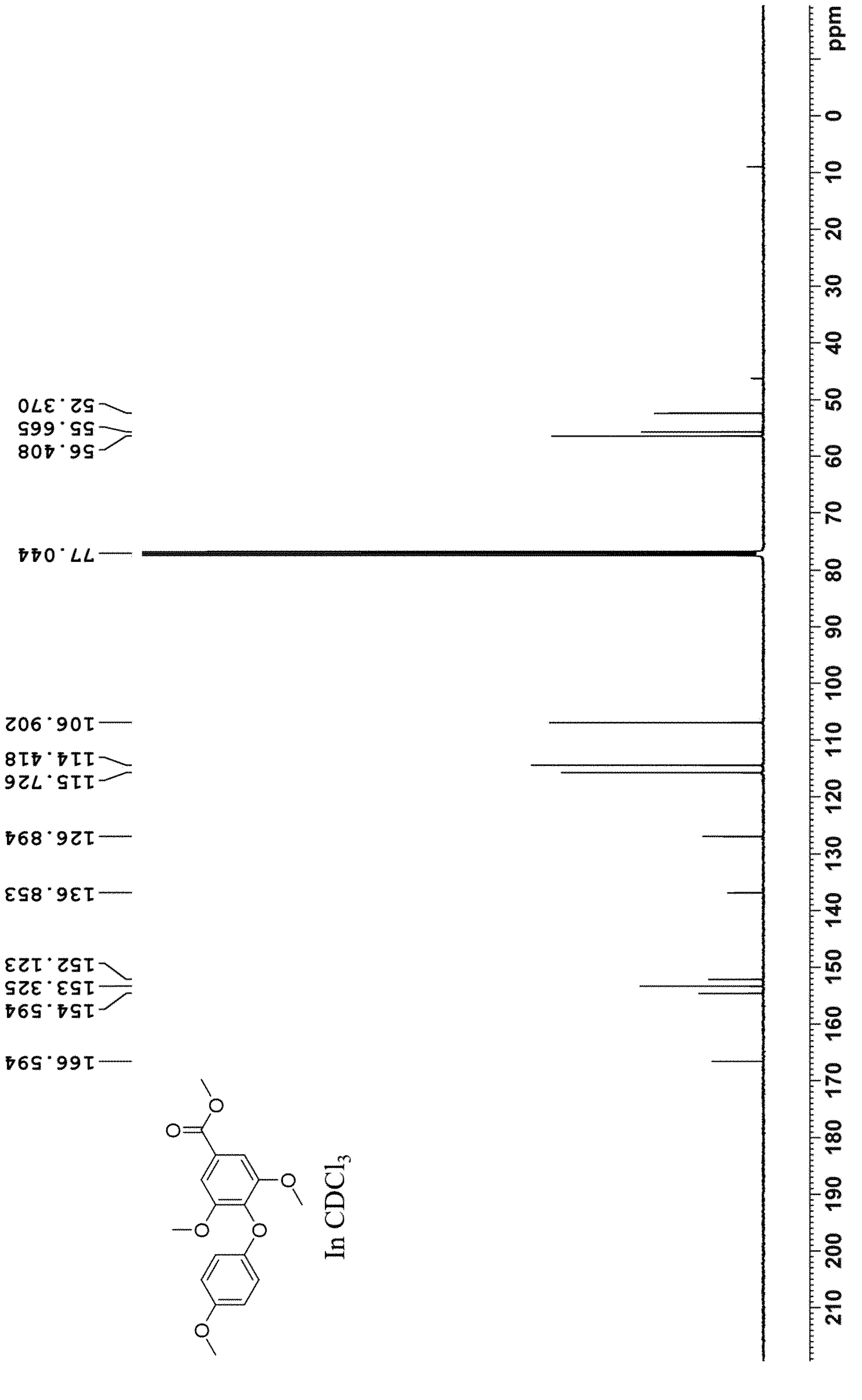
52.370
55.665
56.408
77.044
106.902
114.418
115.726
126.894
136.853
152.123
153.325
154.594
166.594
In CDCl3
ppm
0
10
20
30
40
50
60
70
80
90
100
110
120
130
140
150
160
170
180
190
200
210

Fig.14

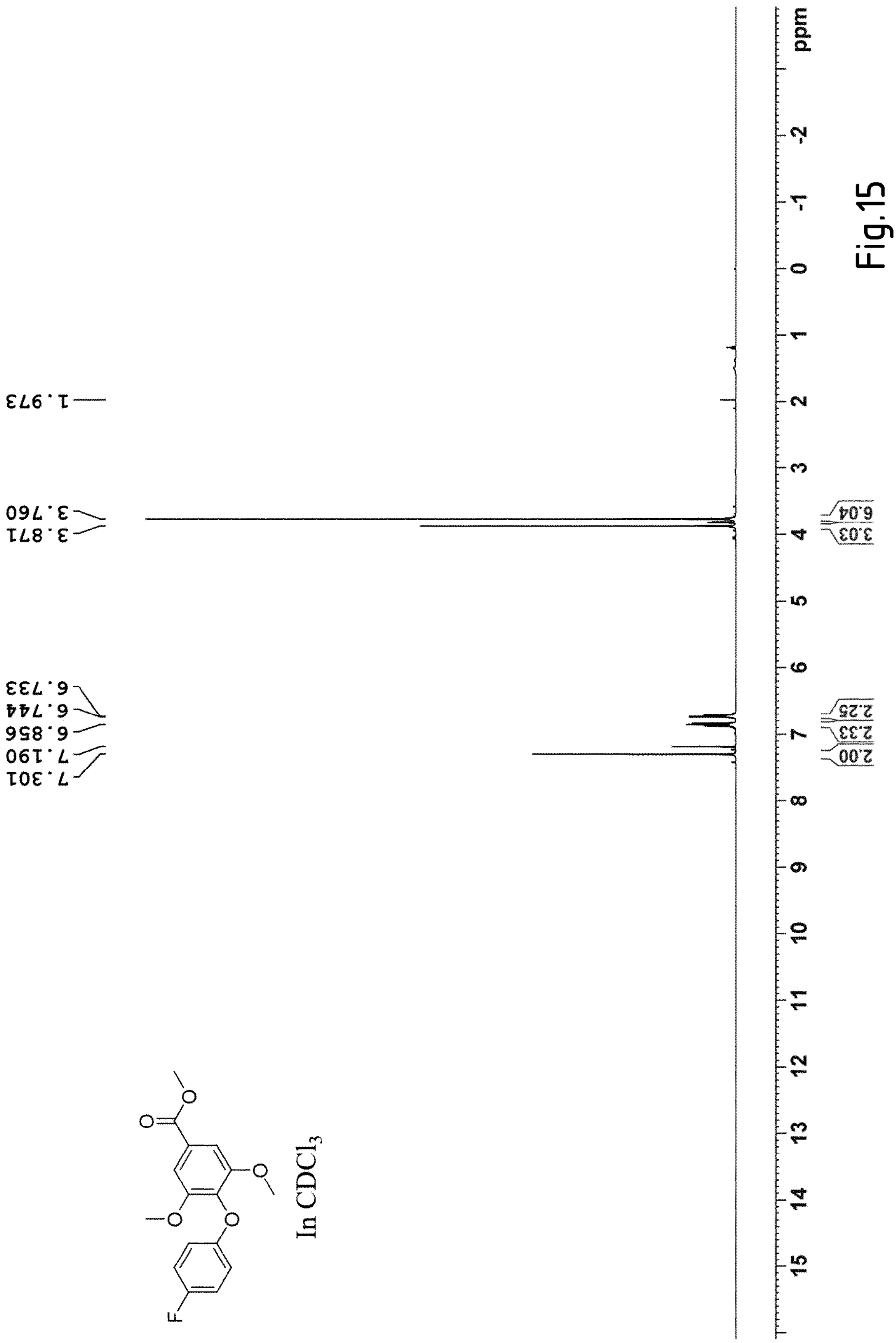
1.973
3.760
3.871
6.733
6.744
6.856
7.190
7.301
6.04
3.03
2.25
2.33
2.00
ppm
In $CDCl_3$

Fig.15

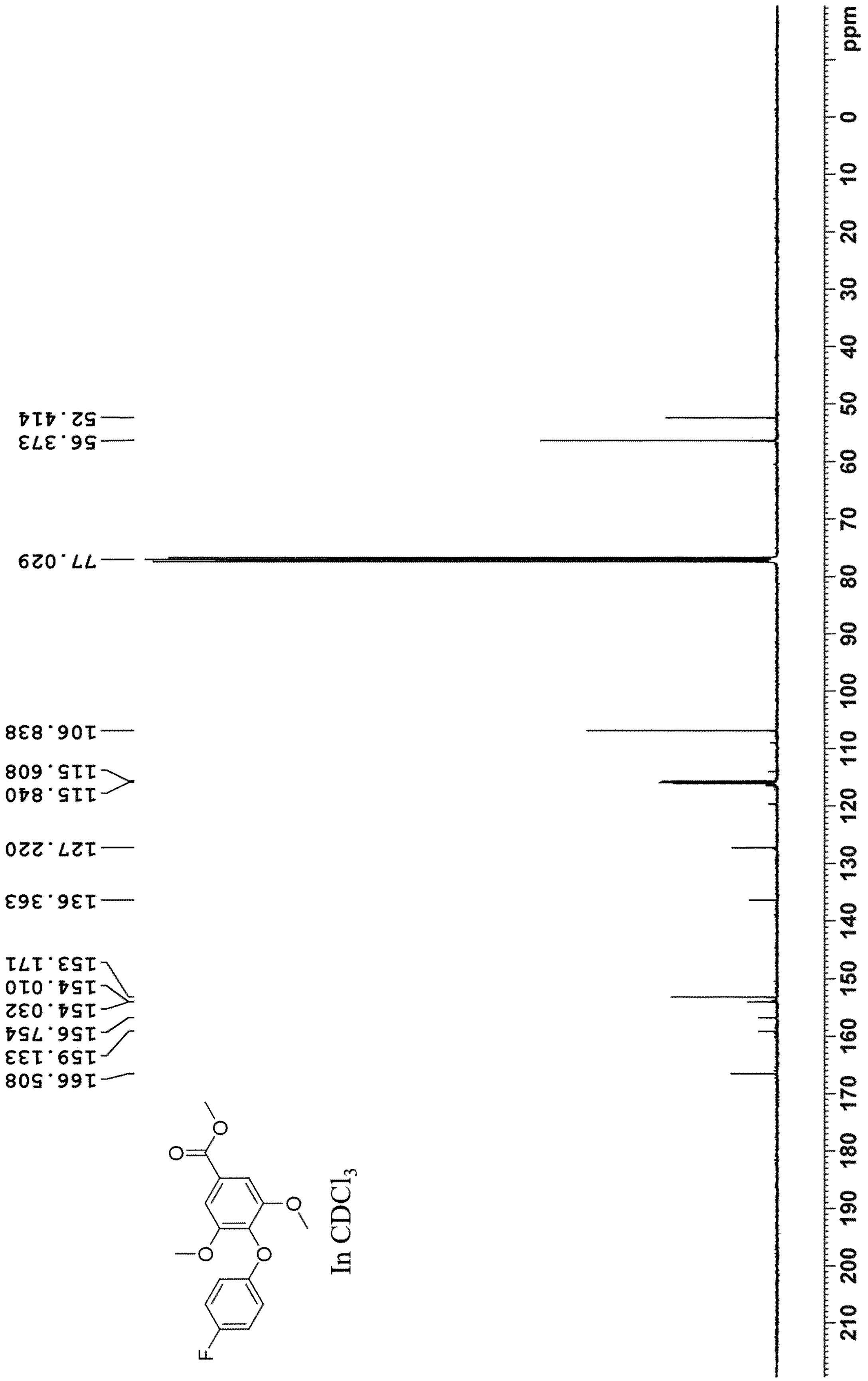

52.414
56.373
77.029
106.838
115.608
115.840
127.220
136.363
153.171
154.010
154.032
156.754
159.133
166.508
In CDCl3
ppm
0
10
20
30
40
50
60
70
80
90
100
110
120
130
140
150
160
170
180
190
200
210


Fig.16

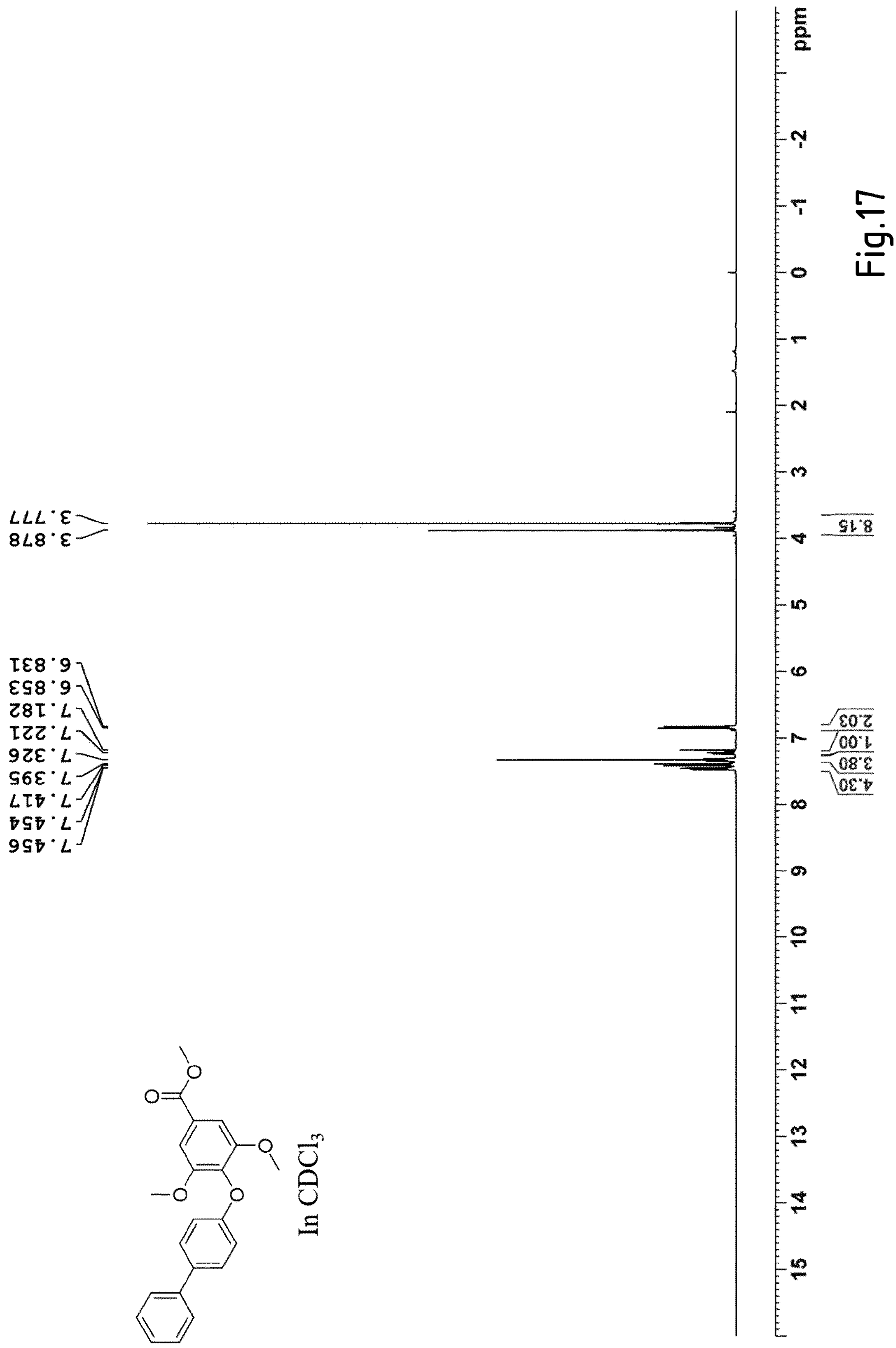
3.777
3.878
6.831
6.853
7.182
7.221
7.326
7.395
7.417
7.454
7.456
In $CDCl_3$
8.15
2.03
1.00
3.80
4.30
ppm

Fig.17

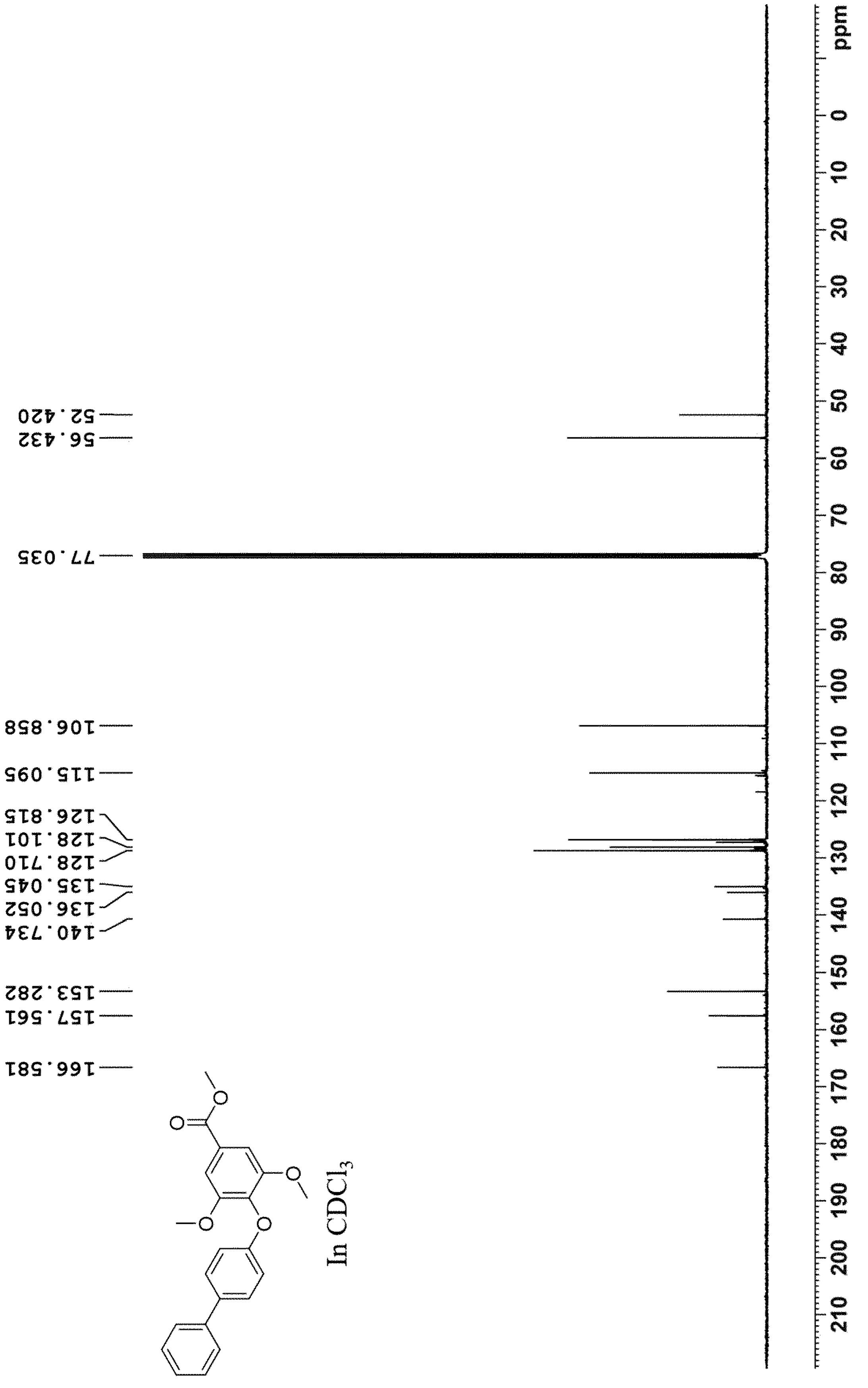
52.420
56.432
77.035
106.858
115.095
126.815
128.101
128.710
135.045
136.052
140.734
153.282
157.561
166.581
In CDCl3
ppm

Fig.18

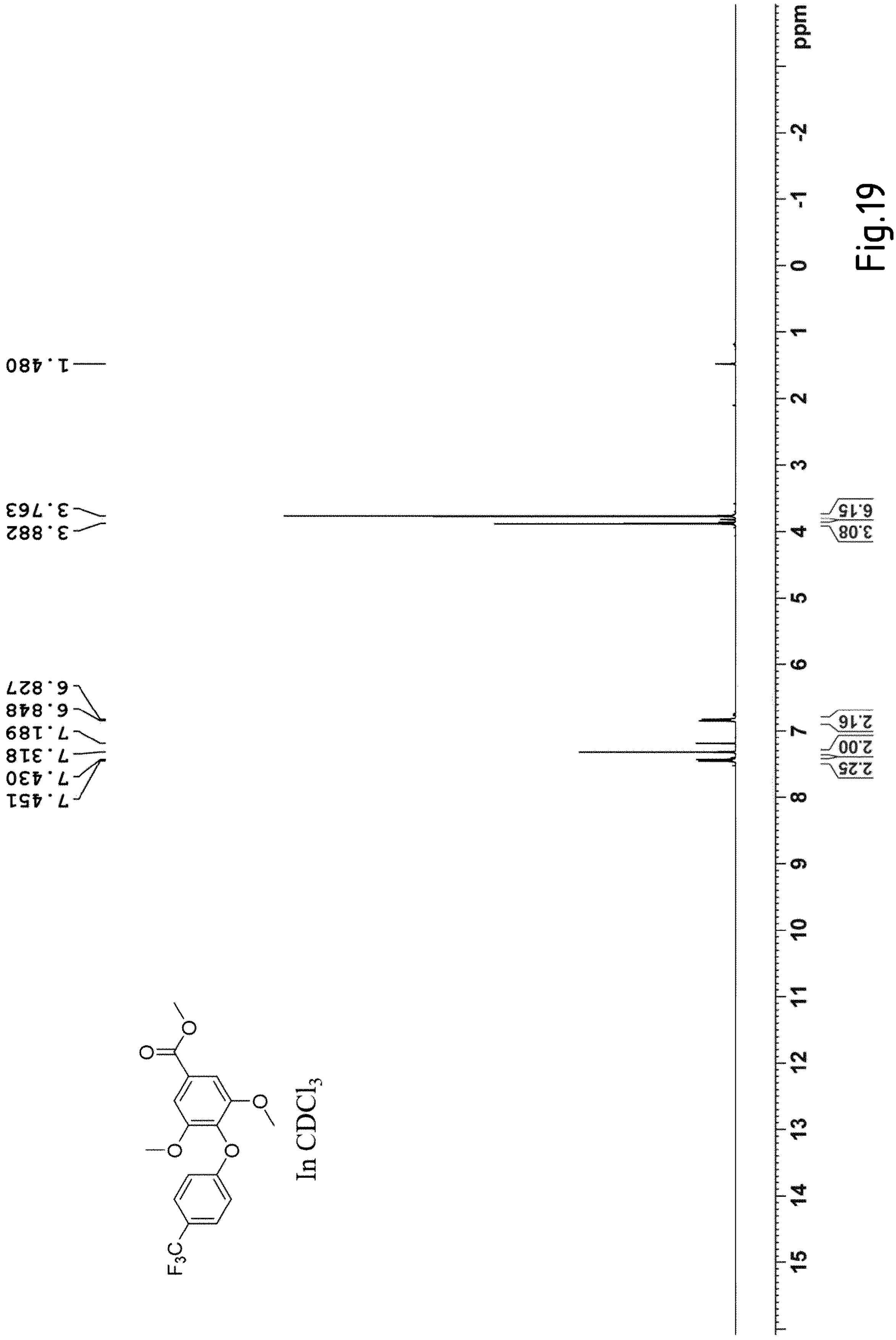

1.480
3.763
3.882
6.827
6.848
7.189
7.318
7.430
7.451
6.15
3.08
2.16
2.00
2.25
In CDCl3
ppm
-2
-1
0
1
2
3
4
5
6
7
8
9
10
11
12
13
14
15


Fig.19

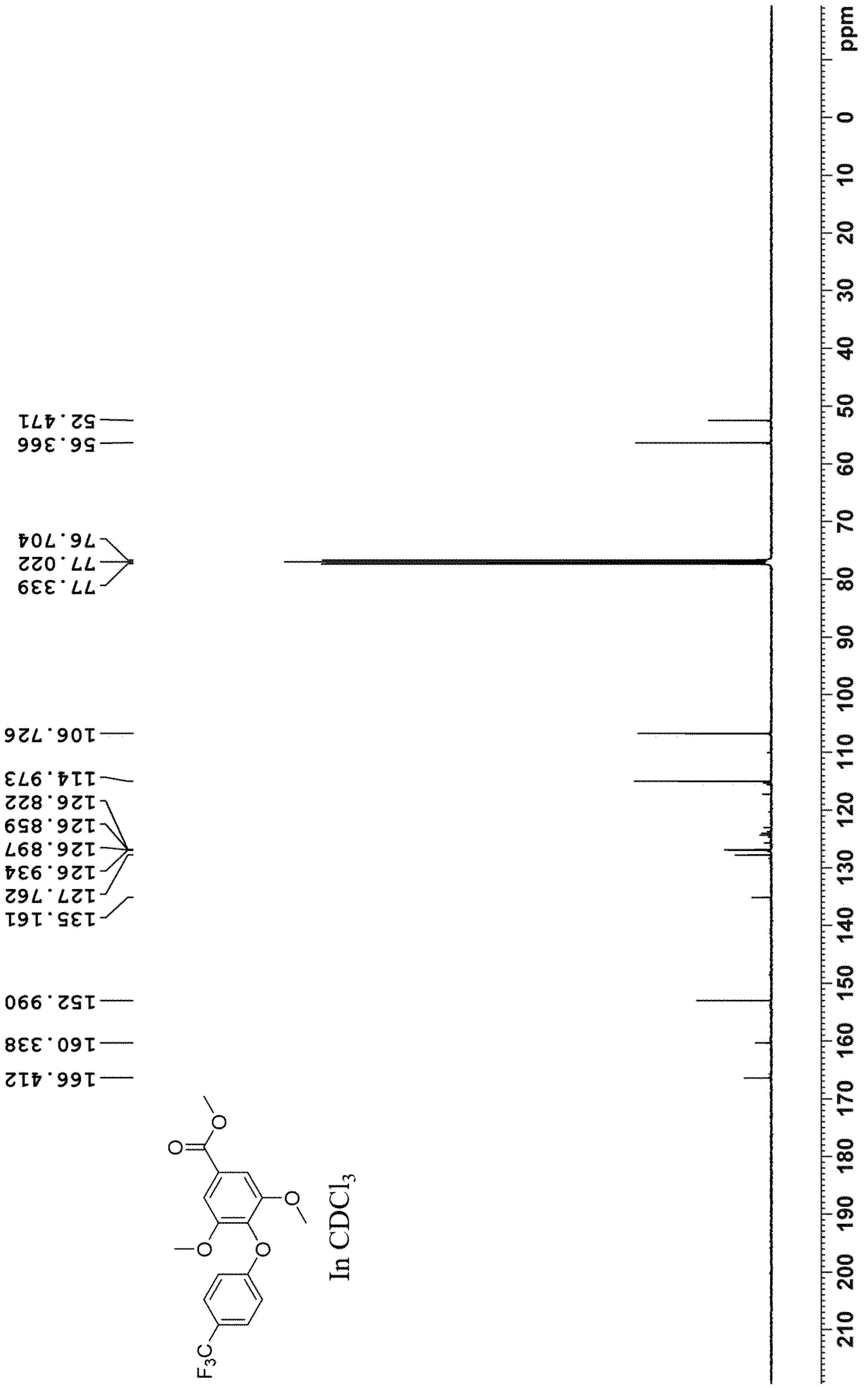
52.471
56.366
76.704
77.022
77.339
106.726
114.973
126.822
126.859
126.897
126.934
127.762
135.161
152.990
160.338
166.412
In $CDCl_3$
ppm
0
10
20
30
40
50
60
70
80
90
100
110
120
130
140
150
160
170
180
190
200
210

Fig.20

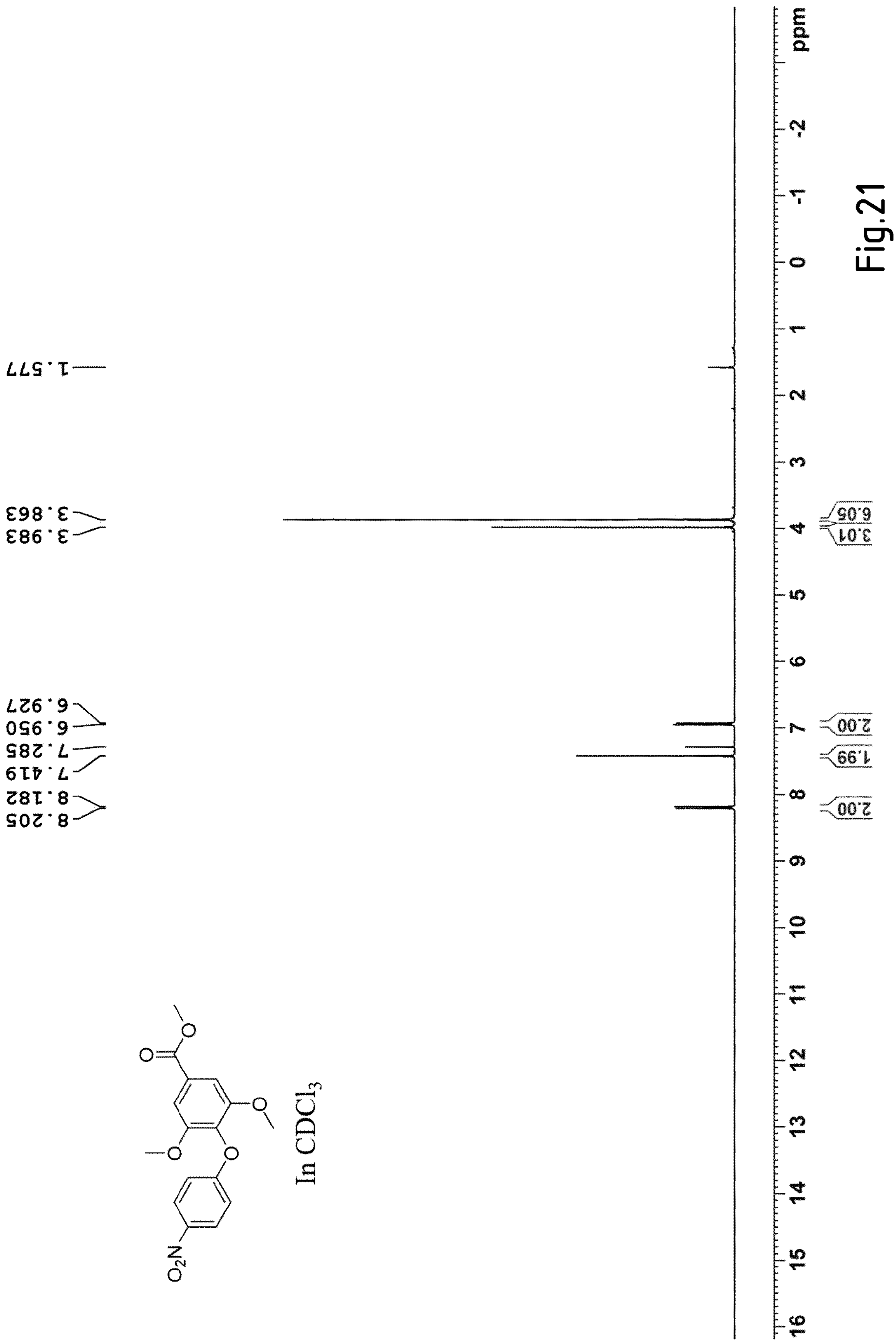

1.577
3.863
3.983
6.927
6.950
7.285
7.419
8.182
8.205
6.05
3.01
2.00
1.99
2.00
In CDCl3
ppm
-2
-1
0
1
2
3
4
5
6
7
8
9
10
11
12
13
14
15
16


Fig.21

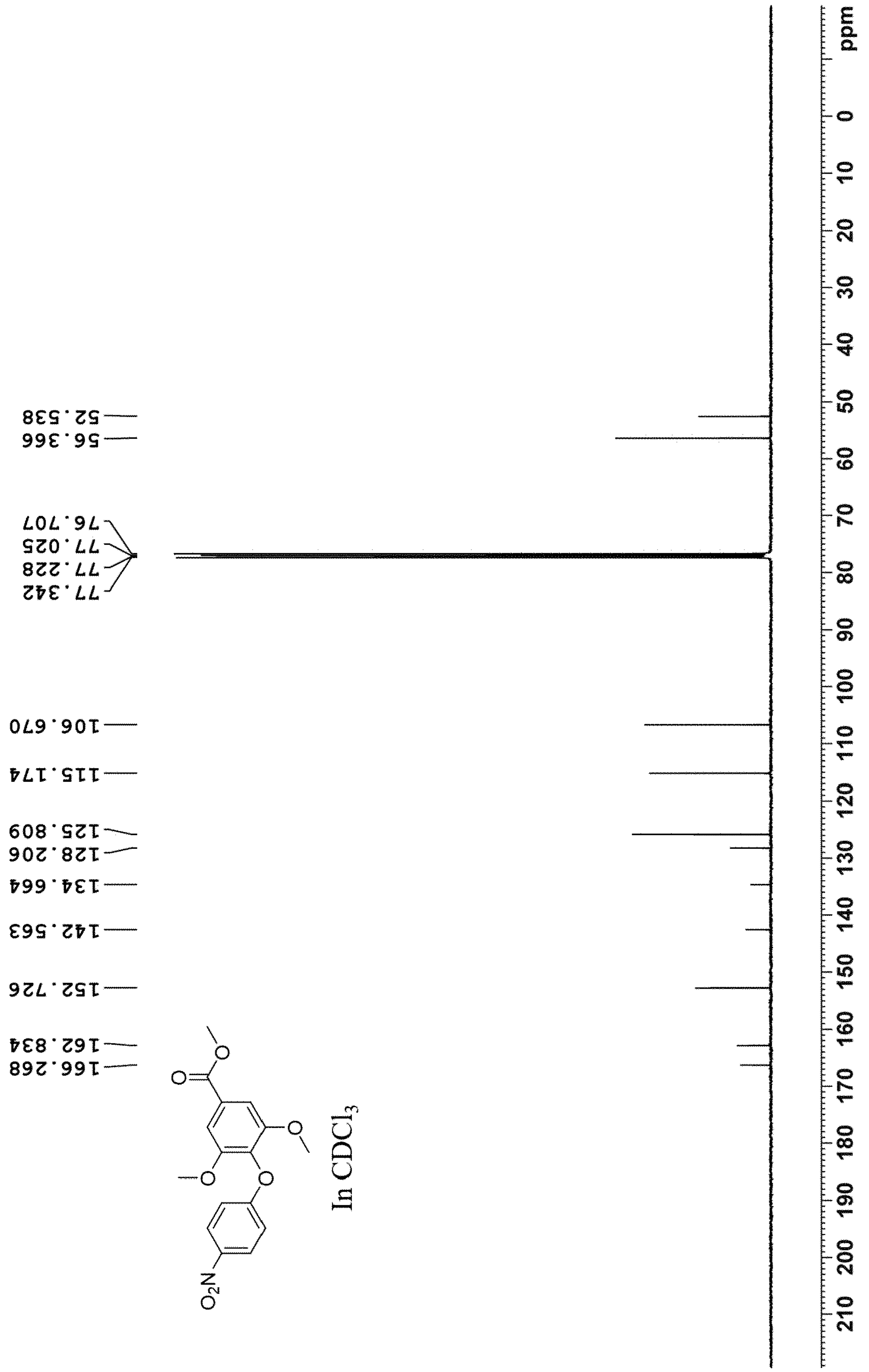
52.538
56.366
76.707
77.025
77.228
77.342
106.670
115.174
125.809
128.206
134.664
142.563
152.726
162.834
166.268
In $CDCl_3$
ppm
0
10
20
30
40
50
60
70
80
90
100
110
120
130
140
150
160
170
180
190
200
210

Fig.22

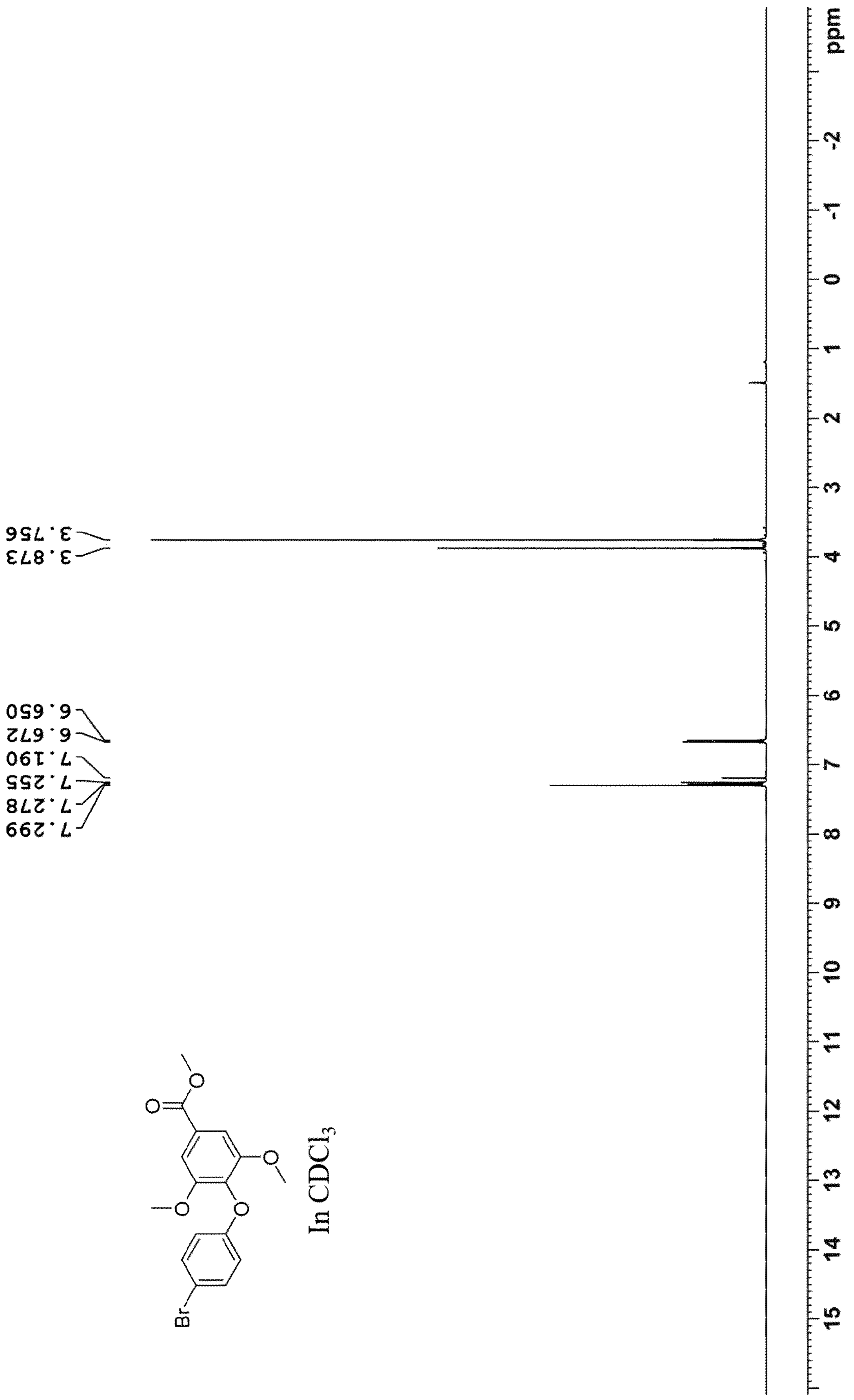
3.756
3.873
6.650
6.672
7.190
7.255
7.278
7.299
6.05
3.02
2.00
4.02
In $CDCl_3$
ppm

Fig.23

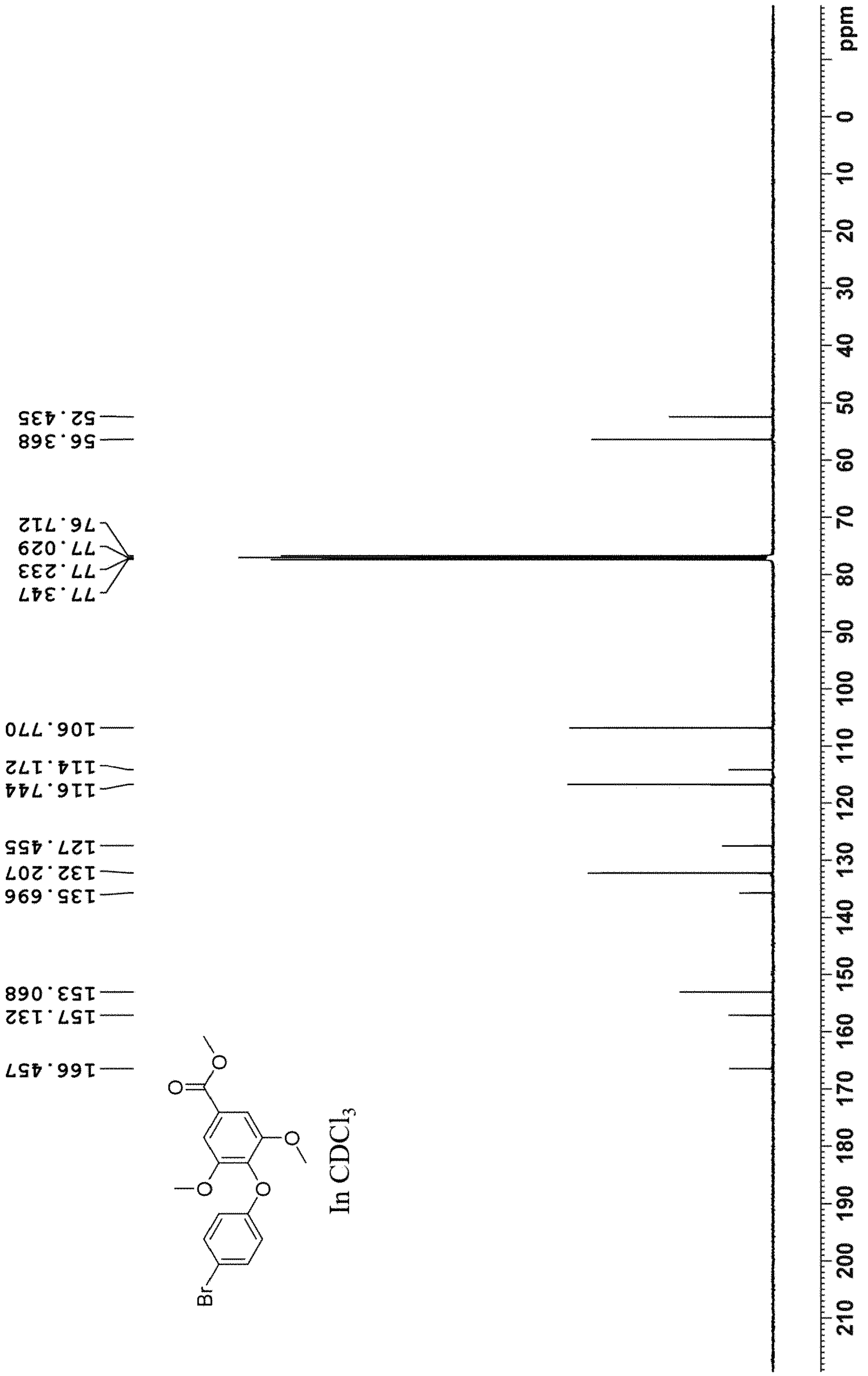
52.435
56.368
76.712
77.029
77.233
77.347
106.770
114.172
116.744
127.455
132.207
135.696
153.068
157.132
166.457
In $CDCl_3$
ppm
0
10
20
30
40
50
60
70
80
90
100
110
120
130
140
150
160
170
180
190
200
210

Fig.24

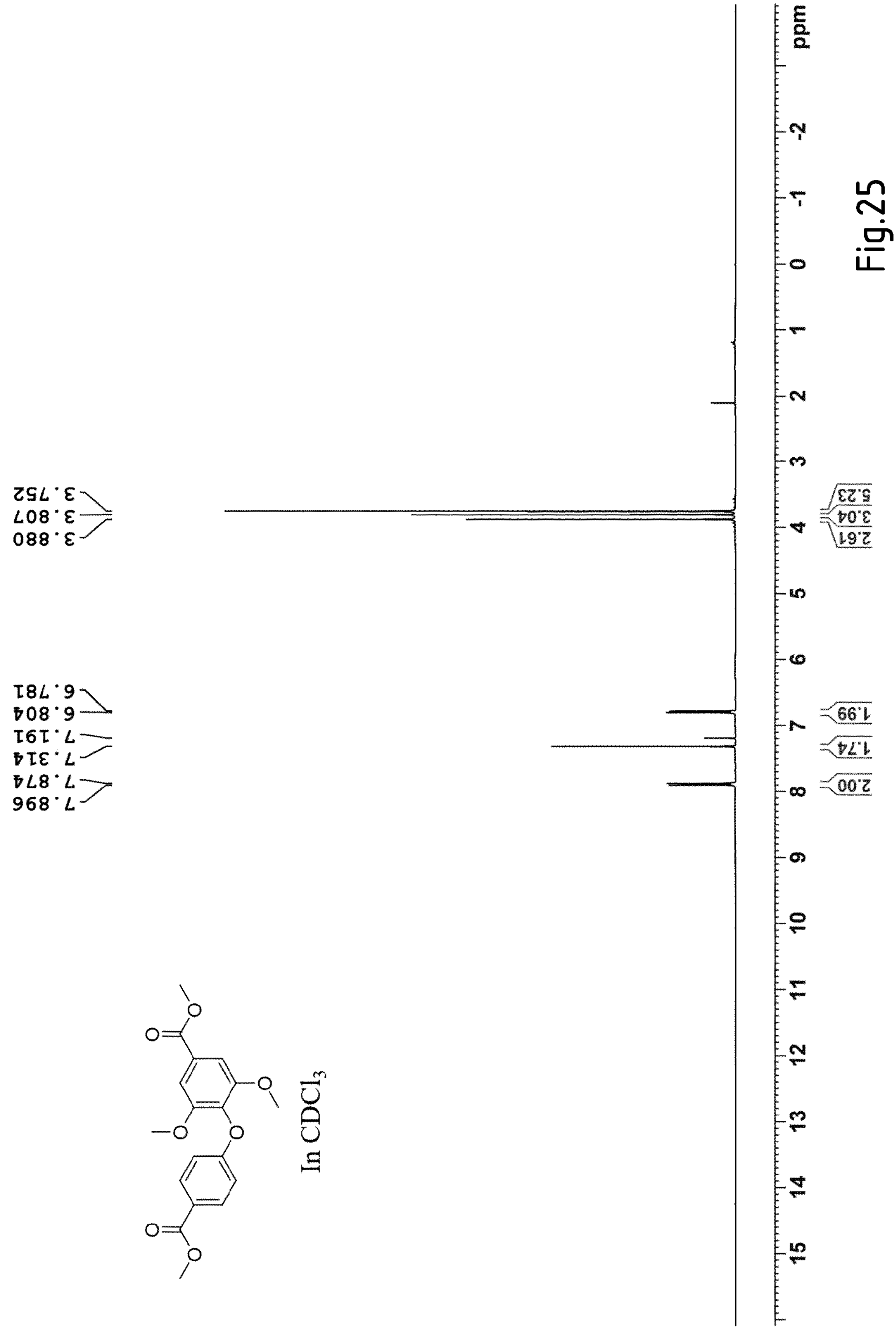
3.752
3.807
3.880
6.781
6.804
7.191
7.314
7.874
7.896
5.23
3.04
2.61
1.99
1.74
2.00
In $CDCl_3$
ppm
-2
-1
0
1
2
3
4
5
6
7
8
9
10
11
12
13
14
15

Fig.25

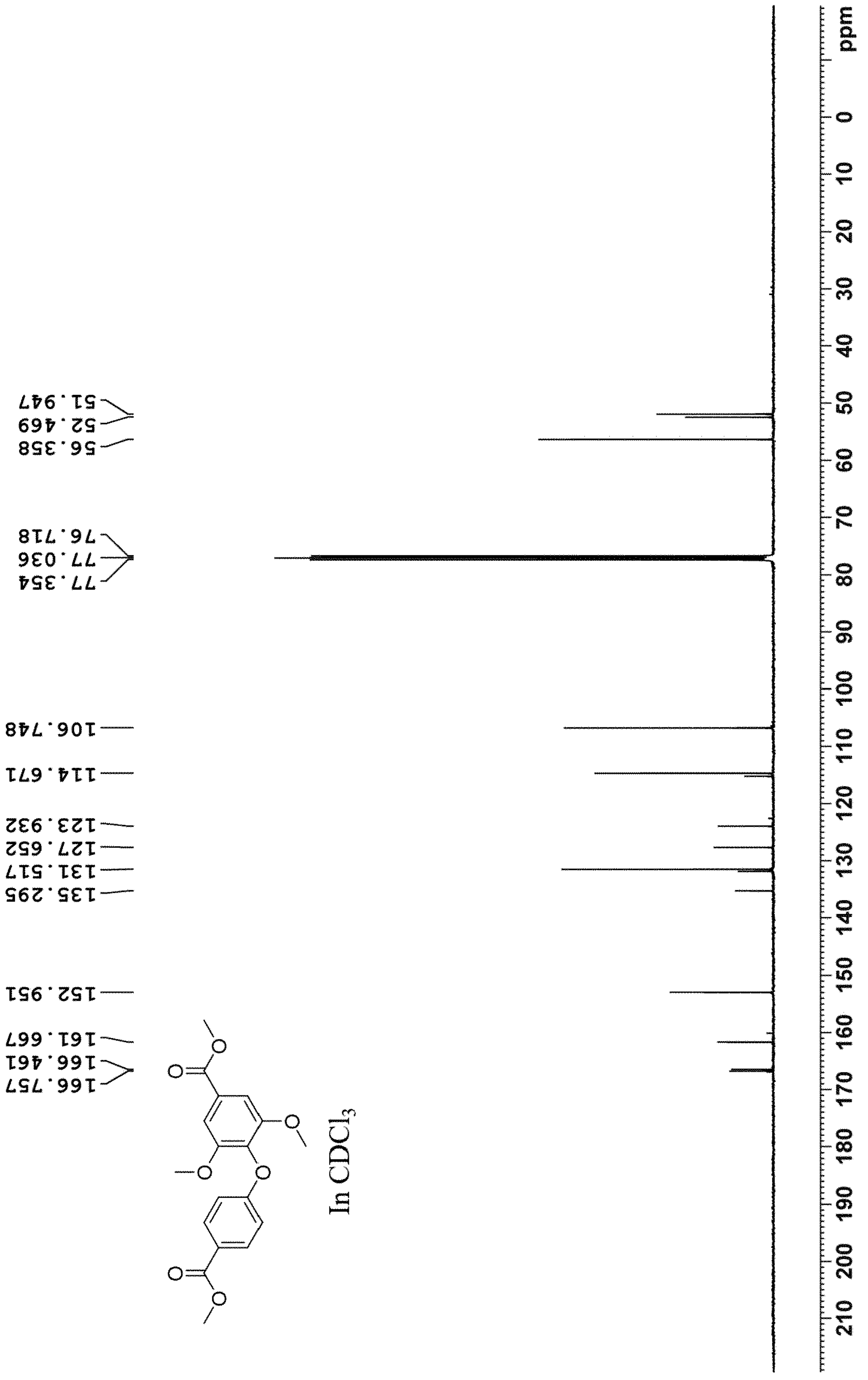
51.947
52.469
56.358
76.718
77.036
77.354
106.748
114.671
123.932
127.652
131.517
135.295
152.951
161.667
166.461
166.757
In CDCl3
ppm

Fig.26

Internal standard:

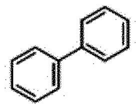

1

Side products from boronic acid:

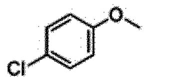

2

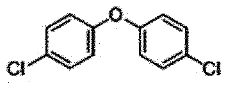

3

Products from lignin:

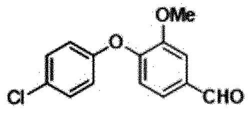

4

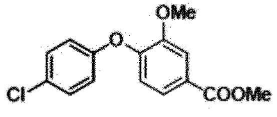

5

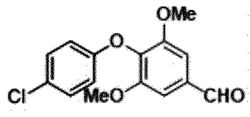

6

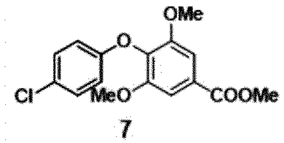

7

1. biphenyl
2. 1-chloro-4-methoxybenzene
3. 4,4'-oxybis(chlorobenzene)
4. 4-(4-chlorophenoxy)-3-methoxybenzaldehyde
5. methyl 4-(4-chlorophenoxy)-3-methoxybenzoate
6. 4-(4-chlorophenoxy)-3,5-dimethoxybenzaldehyde
7. methyl 4-(4-chlorophenoxy)-3,5-dimethoxybenzoate

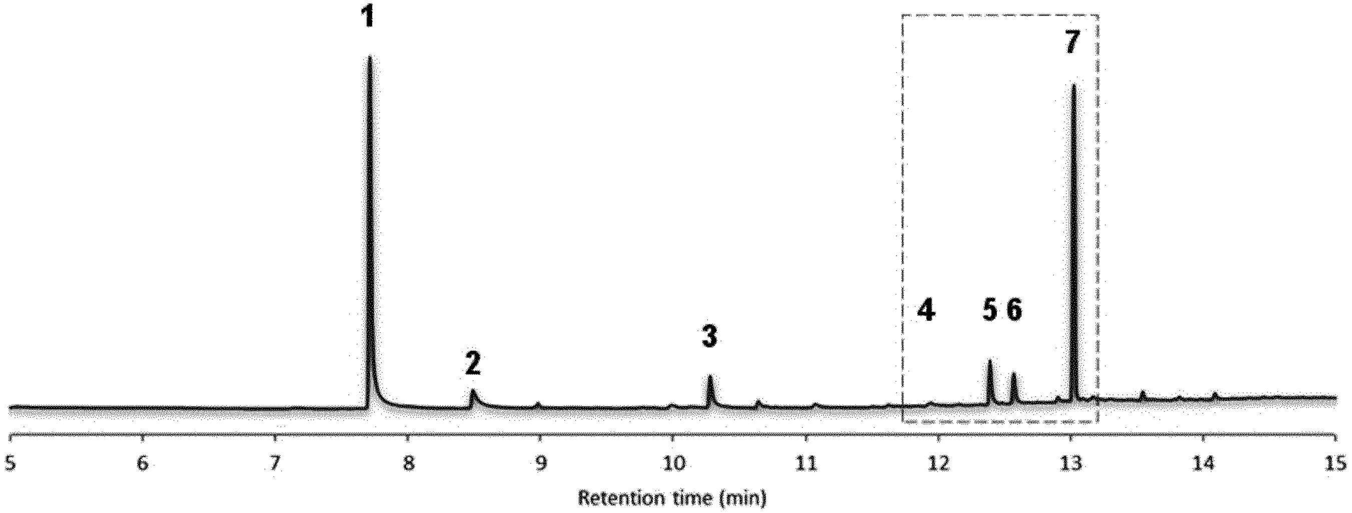


Fig.27

Europäisches Patentamt
European Patent Office
Office européen des brevets

# EUROPEAN SEARCH REPORT

Application Number
EP 22 17 8410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Ghaus Ghulam ET AL: "ENVIRONMENTAL PHOTOINCORPORATIONS OF POLYCHLOROBENZENES INTO SEVERAL HUMIC MODEL MONOMERS ~", Chemosphere, 1 January 1987 (1987-01-01), pages 495-504, XP055975849, DOI: 10.1016/0045-6535(87)90257-8 Retrieved from the Internet: URL:https://www.sciencedirect.com/science/article/pii/0045653587902578 [retrieved on 2022-10-28] * page 500, table, row Products; 2nd and 3rd entry from the bottom * ----- | 1-3,5 | INV. C07C47/575 C07C69/92 |
| X | FELDMAN KEN S. ET AL: "Galloyl-Derived Orthoquinones as Reactive Partners in Nucleophilic Additions and Diels-Alder Dimerizations:? A Novel Route to the Dehydrodigalloyl Linker Unit of Agrimoniin-Type Ellagitannins", THE JOURNAL OF ORGANIC CHEMISTRY, vol. 61, no. 19, 1 January 1996 (1996-01-01), pages 6656-6665, XP055975852, ISSN: 0022-3263, DOI: 10.1021/jo961043u * page 6664, compounds 38, 41, 40 * ----- -/-- | 1-4 | |
| | | | TECHNICAL FIELDS SEARCHED (IPC) |
| | | | C07C |

The present search report has been drawn up for all claims

| Place of search | Date of completion of the search | Examiner |
|---|---|---|
| Munich | 28 October 2022 | Kleidernigg, Oliver |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 22 17 8410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SECA ANA M. L. ET AL: "Structural Characterization of the Bark and Core Lignins from Kenaf ( Hibiscus cannabinus )", JOURNAL OF AGRICULTURAL AND FOOD CHEMISTRY, vol. 46, no. 8, 17 August 1998 (1998-08-17), pages 3100-3108, XP055975853, US ISSN: 0021-8561, DOI: 10.1021/jf9801320 * page 3106, compounds VIII and IX * ----- | 1-3,5 | |
| X | FRADINHO D.M. ET AL: "Chemical characterisation of bark and of alkaline bark extracts from maritime pine grown in Portugal", INDUSTRIAL CROPS AND PRODUCTS, vol. 16, no. 1, 1 July 2002 (2002-07-01), pages 23-32, XP055975854, NL ISSN: 0926-6690, DOI: 10.1016/S0926-6690(02)00004-3 * page 30, compound IX * ----- | 1-3,5 | TECHNICAL FIELDS SEARCHED (IPC) |
| X | PAULA C. PINTO ET AL: "Chemical Composition and Structural Features of the Macromolecular Components of Plantation Acacia mangium Wood", JOURNAL OF AGRICULTURAL AND FOOD CHEMISTRY, vol. 53, no. 20, 1 October 2005 (2005-10-01), pages 7856-7862, XP055108016, ISSN: 0021-8561, DOI: 10.1021/jf058081b * Table 2, compound 9 * ----- -/-- | 1-3,5 | |

The present search report has been drawn up for all claims

1

| Place of search | Date of completion of the search | Examiner |
|---|---|---|
| Munich | 28 October 2022 | Kleidernigg, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 22 17 8410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OLIVEIRA L ET AL: "Structural characterization of stalk lignin from banana plant", INDUSTRIAL CROPS AND PRODUCTS, ELSEVIER, NL, vol. 29, no. 1, 1 January 2009 (2009-01-01), pages 86-95, XP025712753, ISSN: 0926-6690, DOI: 10.1016/J.INDCROP.2008.04.012 [retrieved on 2008-06-06] * figure 5, compounds 8 and 9 * ----- | 1-3,5 | |
| X | PROZIL SÓNIA O. ET AL: "Structural Characterization of Lignin from Grape Stalks ( Vitis vinifera L.)", JOURNAL OF AGRICULTURAL AND FOOD CHEMISTRY, vol. 62, no. 24, 3 June 2014 (2014-06-03), pages 5420-5428, XP055975856, US ISSN: 0021-8561, DOI: 10.1021/jf502267s * figure 2, compound 13 * ----- | 1-3,5 | |
| | -/-- | | TECHNICAL FIELDS SEARCHED (IPC) |

The present search report has been drawn up for all claims

1

| Place of search | Date of completion of the search | Examiner |
|---|---|---|
| Munich | 28 October 2022 | Kleidernigg, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 17 8410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Wünsche Ch ET AL: "ELEKTRONENSTOSS-INDUZIERTE ALKYL-UND WASSERSTOFFWANDERUNG-II'", Tetrahedron, 1 January 1969 (1969-01-01), pages 73-87, XP055975860, Retrieved from the Internet: URL:https://reader.elsevier.com/reader/sd/pii/S004040200199458X?token=0F24BC12D1BB0B46322464F5E9367764543146CB7CB60AA7A3AEEF39549107B25BBFB6C850208F872089B2532635D063&originRegion=eu-west-1&originCreation=20221028102940 [retrieved on 2022-10-28] * table 11 * ----- | 1-5 | |
| A,D | CHRISTOPHER S. LANCEFIELD ET AL: "Isolation of Functionalized Phenolic Monomers through Selective Oxidation and CO Bond Cleavage of the &bgr;-O-4 Linkages in Lignin", ANGEWANDTE CHEMIE INTERNATIONAL EDITION, vol. 54, no. 1, 2 January 2015 (2015-01-02), pages 258-262, XP055215211, ISSN: 1433-7851, DOI: 10.1002/anie.201409408 * Scheme 1; figure 1; table 1 * ----- | 1-14 | |
| | | | TECHNICAL FIELDS SEARCHED (IPC) |

1

The present search report has been drawn up for all claims

| Place of search | Date of completion of the search | Examiner |
|---|---|---|
| Munich | 28 October 2022 | Kleidernigg, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RAGAUSKAS A. J. ; BECKHAM G. T. ; BIDDY M. J. ; CHANDRA R. ; CHEN F. ; DAVIS M. F. ; DAVISON B. H. ; DIXON R. A. ; GILNA P. ; KELLER M.** *Science,* 2014, vol. 344, 1246843
- **RINALDI R. ; JASTRZEBSKI R. ; CLOUGH M. T. ; RALPH J. ; KENNEMA M. ; BRUIJNINCX P. C. A. ; WECKHUYSEN B. M.** *Angew. Chem. Int. Ed.,* 2016, vol. 55, 8164-8215
- **SUN Z. ; FRIDRICH B. ; DE SANTI A. ; ELANGOVAN S. ; BARTA K.** *Chem. Rev.,* 2018, vol. 118, 614-678
- **SCHUTYSER W. ; RENDERS T. ; VAN DEN BOSCH S. ; KOELEWIJN S. F. ; BECKHAM G. T. ; SELS B. F.** *Chem. Soc. Rev.,* 2018, vol. 47, 852-908
- **QUESTELL-SANTIAGO Y. M. ; GALKIN M. V. ; BARTA K. ; LUTERBACHER J. S.** *Nat. Rev. Chem.,* 2020, vol. 4, 311-330
- **ABU-OMAR M. M. ; BARTA K. ; BECKHAM G. T. ; LUTERBACHER J. S. ; RALPH J. ; RINALDI R. ; ROMAN-LESHKOV Y. ; SAMEC J. S. M. ; SELS B. F. ; WANG F.** *Energy Environ. Sci.,* 2021, vol. 14, 262-292
- **BEHLING R. ; VALANGE S. ; CHATEL G.** *Green Chem.,* 2016, vol. 18, 1839-1854
- **YANG C. ; MALDONADO S. ; STEPHENSON C. R. J.** *ACS Catal.,* 2021, vol. 11, 10104-10114
- **CAI Z. ; LONG J. ; LI Y. ; YE L. ; YIN B. ; FRANCE L. J. ; DONG J. ; ZHENG L. ; HE H. ; LIU S.** *Chem,* 2019, vol. 5, 2365-2377
- **LUO H. ; WEEDA E. P. ; ALHERECH M. ; ANSON C. W. ; KARLEN S. D. ; CUI Y. ; FOSTER C. E. ; STAHL S. S.** *J. Am. Chem. Soc.,* 2021, vol. 143, 15462-15470
- **SUBBOTINA E. ; RUKKIJAKAN T. ; MARQUEZ-MEDINA M. D. ; YU X. ; JOHNSSON M. ; SAMEC J. S. M.** *Nat. Chem.,* 2021, vol. 13, 1118-1125
- **SEDAI B. ; BAKER R. T.** *Adv. Synth. Catal.,* 2014, vol. 356, 3563-3574
- **WANG M. ; LU J. ; LI L. ; LI H. ; LIU H. ; WANG F.** *J. Catal.,* 2017, vol. 348, 160-167
- **SCHUTYSER W. ; KRUGER J. S. ; ROBINSON A. M. ; KATAHIRA R. ; BRANDNER D. G. ; CLEVELAND N. S. ; MITTAL A. ; PETERSON D. J. ; MEILAN R. ; ROMÁN-LESHKOV Y.** *Green Chem.,* 2018, vol. 20, 3828-3844
- **LIU M. ; ZHANG Z. ; SHEN X. ; LIU H. ; ZHANG P. ; CHEN B. ; HAN B.** *Chem. Commun.,* 2019, vol. 55, 925-928
- **LIU M. ; ZHANG Z. ; SONG J. ; LIU S. ; LIU H. ; HAN B.** *Angew. Chem. Int. Ed.,* 2019, vol. 58, 17393-17398
- **LIU J. ; QIU X. ; HUANG X. ; LUO X. ; ZHANG C. ; WEI J. ; PAN J. ; LIANG Y. ; ZHU Y. ; QIN Q.** *Nat. Chem.,* 2019, vol. 11, 71-77
- **LIU M. ; ZHANG Z. ; YAN J. ; LIU S. ; LIU H. ; LIU Z. ; WANG W. ; HE Z. ; HAN B.** *Chem,* 2020, vol. 6, 3288-3296
- **LUO H. ; WANG L. ; SHANG S. ; LI G. ; LV Y. ; GAO S. ; DAI W.** *Angew. Chem. Int. Ed.,* 2020, vol. 59, 19268-19274
- **ZHOU H. ; LI Z. ; XU S.-M. ; LU L. ; XU M. ; JI K. ; GE R. ; YAN Y. ; MA L. ; KONG X.** *Angew. Chem. Int. Ed.,* 2021, vol. 60, 8976-8982
- **CUI T. ; MA L. ; WANG S. ; YE C. ; LIANG X. ; ZHANG Z. ; MENG G. ; ZHENG L. ; HU H.-S. ; ZHANG J.** *J. Am. Chem. Soc.,* 2021, vol. 143, 9429-9439
- **MA L. ; ZHOU H. ; KONG X. ; LI Z. ; DUAN H.** *ACS Sustain. Chem. Eng.,* 2021, vol. 9, 1932-1940
- **SHI S.-H. ; LIANG Y. ; JIAO N.** *Chem. Rev.,* 2021, vol. 121, 485-505
- **RAHIMI A. ; ULBRICH A. ; COON J. J. ; STAHL S. S.** *Nature,* 2014, vol. 515, 249-252
- **ESGUERRA K. V. N. ; FALL Y. ; PETITJEAN L. ; LUMB J.-P.** *J. Am. Chem. Soc.,* 2014, vol. 136, 7662-7668
- **NIEDERER K. A. ; GILMARTIN P. H. ; KOZLOWSKI M. C.** *ACS Catal.,* 2020, vol. 10, 14615-14623
- **XU W. ; HUANG Z. ; JI X. ; LUMB J.-P.** *ACS Catal.,* 2019, vol. 9, 3800-3810
- **NEUHAUS W. C. ; KOZLOWSKI M. C.** *Angew. Chem. Int. Ed.,* 2020, vol. 59, 7842-7847
- **LANCEFIELD, C. S. ; OJO, O. S. ; TRAN, F. ; WESTWOOD, N. J.** Isolation of Functionalized Phenolic Monomers through Selective Oxidation and CO Bond Cleavage of the β-O-4 Linkages in Lignin. *Angew. Chem. Int. Ed.,* 2015, vol. 54, 258-262
- **SETTE M. ; LANGE H. ; CRESTINI C.** QUANTITATIVE HSQC ANALYSES OF LIGNIN: A PRACTICAL COMPARISON. *Comput. Struct. Biotechnol. J.,* 2013, vol. 6

- **HEIKKINEN, S. ; TOIKKA, M. M. ; KARHUNEN, P. T. ; KILPELÄINEN, I. A.** Quantitative 2D HSQC (Q-HSQC) via Suppression of J-Dependence of Polarization Transfer in NMR Spectroscopy: Application to Wood Lignin. *J. Am. Chem. Soc.,* 2003, vol. 125, 4362-4367
- **HEIKKINEN, S. ; TOIKKA, M. M. ; KARHUNEN, P. T. ; KILPELÄINEN, I. A.** Quantitative 2D HSQC (Q-HSQC) via Suppression of J-Dependence of Polarization Transfer in NMR Spectroscopy: Application to Wood Lignin. *J.Am.Chem.Soc.,* 2003, vol. 125, 4362-4367